# EUROPEAN PATENT APPLICATION

(11) **EP 4 806 459 A1**
(43) Date of publication of application: **16.09.2026**
(21) Application number: 24888113.8
(22) Date of filing: 08.11.2024
(51) Int. Cl.: A61K 38/28, C07K 14/62, A61K 47/54, A61P 3/10

(54) **THERAPEUTIC USE OF PHARMACEUTICAL COMPOSITION COMPRISING INSULIN DERIVATIVE**

(30) Priority: 08.11.2023 CN 202311480982; 02.06.2024 CN 202410702342; 09.08.2024 CN 202411090791
(71) Applicant: GAN & LEE PHARMACEUTICALS CO., LTD., Beijing 101109 (CN)
(72) Inventor: GAN, Zhongru, Beijing 101109 (CN); HAO, Chunyue, Beijing 101109 (CN); ZHAO, Jing, Beijing 101109 (CN); CHEN, Wei, Beijing 101109 (CN)
(74) Representative: Kraus & Lederer PartGmbB
(86) International application number: PCT/CN2024/131090
(87) International publication number: WO 2025/098502

(57) **Abstract**

The present invention relates to the use of a pharmaceutical combination or pharmaceutical composition containing two active ingredients, an insulin derivative and a rapid-acting insulin, in the treatment of diabetes mellitus. Provided is a method for treating or preventing metabolic syndrome and type II diabetes in a subject in need thereof by administering a compound of formula (I) or a pharmaceutically acceptable salt thereof. Also provided is a kit comprising the description for performing treatment according to the method.

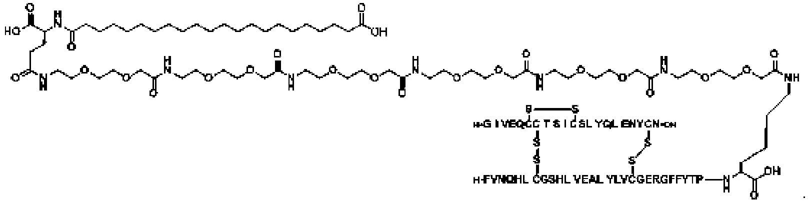

## Description

The present application claims priority to Chinese Patent Application No. 202311480982.4 filed on November 8, 2023, Chinese Patent Application No. 202410702342.1 filed on June 2, 2024, and Chinese Patent Application No. 202411090791.1 filed on August 9, 2024. The entire contents of the above three applications are incorporated herein by reference.

### TECHNICAL FIELD

The present invention relates to the field of pharmaceutical uses of drugs, specifically to the use of a drug comprising two active ingredients, an insulin derivative and a rapid-acting insulin, in the treatment of type II diabetes.

### BACKGROUND

Diabetes is a metabolic disorder characterized by hyperglycemia. Long-term hyperglycemia leads to chronic damage and functional impairment in various tissues, particularly the eyes, kidneys, heart, blood vessels, and nerves. Among diabetes patients, the majority suffer from type 2 diabetes. Although several oral antidiabetic drugs and insulin are currently available for treatment, some patients still experience suboptimal clinical outcomes with oral pharmaceuticals and insulin therapy. Consequently, the need for effective glycemic control drugs in these patients remains unmet.

### SUMMARY

In some embodiments, the present invention relates to a method of treating type II diabetes, comprising administering a therapeutically effective amount of a pharmaceutical composition to an adult patient with type II diabetes inadequately controlled by oral antidiabetic drugs, or to an adult patient with type II diabetes who is inadequately controlled by prior use of basal or premixed insulin, wherein the method can achieve effective glycemic control at a certain dosage regimen. The dosing regimens described herein provide benefits for the treatment of type II diabetes with the compound of formula (I), maximizing its hypoglycemic efficacy while reducing or eliminating adverse side effects caused by its glucose-lowering action.

The disclosure provides method for treating metabolic syndrome, wherein the method comprises administering a combination of a compound of formula (I) and insulin aspart to a subject in need thereof, wherein a total administered dose of the compound of formula (I) and insulin aspart is about 5U-200U, preferably about 5U-50U, preferably the total doses of each administration are independently the same or different; preferably, when administering the combination of the compound of formula (I) and insulin aspart, the compound of formula (I) and insulin aspart are administered as a mixture or administered separately; preferably, when administering the combination of the compound of formula (I) and insulin aspart, the compound of formula (I) and insulin aspart are administered simultaneously; preferably, the combination of the compound of formula (I) and insulin aspart is a pharmaceutical composition comprising the compound of formula (I) and insulin aspart;

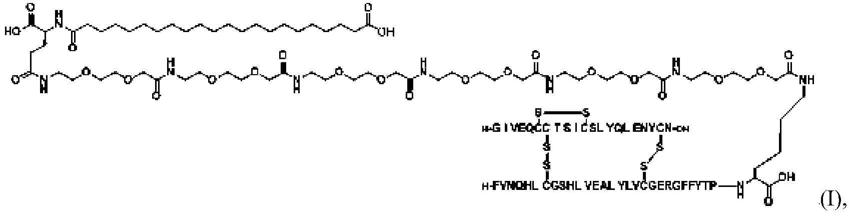

In some embodiments, the metabolic syndrome is diabetes mellitus; preferably, the diabetes mellitus is type II diabetes.

In some embodiments, the method comprises administering a pharmaceutical composition comprising a compound of formula (I) and insulin aspart to a subject in need thereof, wherein the total administered dose of the compound of formula (I) and insulin aspart is 5U-200U, preferably about 5U-50U; preferably, the total doses of each administration are independently the same or different; 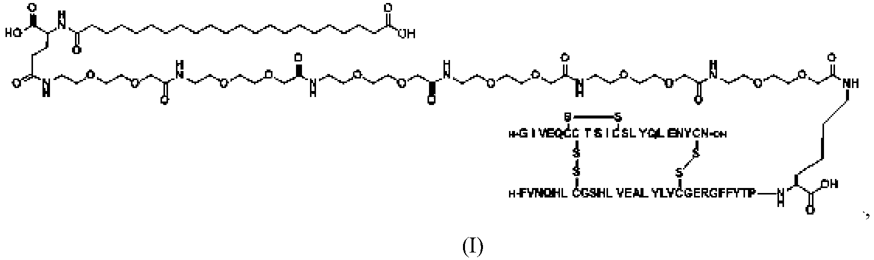 preferably, the metabolic syndrome is diabetes mellitus; preferably, the diabetes mellitus is type II diabetes.

In some embodiments, in the combination of the compound of formula (I) and insulin aspart, and in the pharmaceutical composition comprising the compound of formula (I) and insulin aspart, the molar percentage of the compound of formula (I) in the combination or the pharmaceutical composition is about 50%-99%; preferably, the molar percentage of the compound of formula (I) in the combination or the pharmaceutical composition is about 50%, about 55%, about 60%, about 65%, about 70%, about 75%, about 80%, about 85%, about 90%, or about 95%; preferably, the molar percentage of the compound of formula (I) in the combination or the pharmaceutical composition is about 50%, about 60%, about 70%, or about 80%; preferably, a molar ratio of the compound of formula (I) to insulin aspart is 1:1 or 7:3.

In some embodiments, a total daily dose of the compound of formula (I) and insulin aspart is about 5U-200U, preferably about 5U-50U, preferably about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 55U, about 60U, about 65U, about 70U, about 75U, about 80U, about 85U, about 90U, about 95U, about 100U, about 110U, about 120U, about 130U, about 140U, about 150U, about 160U, about 170U, about 180U, about 190U, or about 200U.
preferably, the total daily doses are independently the same or different.In some embodiments, the method comprises administering the combination of the compound of formula (I) and insulin aspart or the pharmaceutical composition comprising the compound of formula (I) and insulin aspart to the subject in need thereof at a frequency of once daily or less, preferably the total doses of each administration are independently the same or different.In some embodiments, the method comprises administering the compound of formula (I) and insulin aspart to the subject in need thereof at a frequency of once daily or less and a total dose of about 5U-200U, preferably about 5U-50U, as a starting dose, preferably administering the compound of formula (I) and insulin aspart to the subject in need thereof at a frequency of once daily or less and the following total dose as a starting dose: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 55U, about 60U, about 65U, about 70U, about 75U, about 80U, about 85U, about 90U, about 95U, about 100U, about 110U, about 120U, about 130U, about 140U, about 150U, about 160U, about 170U, about 180U, about 190U, or about 200U.

In some embodiments, for a subject previously administered with a basal or premixed insulin once daily, when switching to administering the combination of the compound of formula (I) and insulin aspart or the pharmaceutical composition comprising the compound of formula (I) and insulin aspart, a total starting dose of the compound of formula (I) and insulin aspart is about 50%-90%, preferably about 60%-85%, preferably about 70%-80%, preferably about 50%, about 60%, about 70%, about 75%, or about 80% of the total daily dose of the previous basal or premixed insulin.

In some embodiments, the method further comprises, after administering the compound of formula (I) and insulin aspart to the subject in need thereof at the starting dose, further administering the compound of formula (I) and insulin aspart at a frequency of once daily or less, wherein a total dose of each administration is the same as or different from the starting dose, preferably, the total dose of each administration is determined according to the individual conditions of the subject.

In some embodiments, the method comprises, when administering the combination of the compound of formula (I) and insulin aspart, or the pharmaceutical composition comprising the compound of formula (I) and insulin aspart, the compound of formula (I) and insulin aspart are administered to the subject in need thereof at a frequency of once daily or less and at a total dose of about 5U-200U, preferably about 5U-50U, as the starting dose; preferably the compound of formula (I) and insulin aspart are administered to the subject in need thereof at a frequency of once daily or less and the following total dose is used as the starting dose: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 55U, about 60U, about 65U, about 70U, about 75U, about 80U, about 85U, about 90U, about 95U, about 100U, about 110U, about 120U, about 130U, about 140U, about 150U, about 160U, about 170U, about 180U, about 190U, or about 200U.

In some embodiments, after administering the starting dose for 1 day, 2 days, 3 days, or 4 days, or after administering for 1, 2, 3, 4, 5, 6, 7, 8, 9, 10, 11, 12, 13, 14, 15 or 16 weeks, the compound of formula (I) and insulin aspart are administered at a frequency of once daily and at a total dose of M, wherein the dose M administered each day is the same or different, and is independently about 1U-200U, preferably about 1U-70U, preferably is independently about 1U, about 2U, about 3U, about 4U, about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 55U, about 60U, about 65U, about 70U, about 75U, about 80U, about 85U, about 90U, about 95U, about 100U, about 110U, about 120U, about 130U, about 140U, about 150U, about 160U, about 170U, about 180U, about 190U, or about 200U.

In some embodiments, the method comprises administering the compound of formula (I) and insulin aspart to the subject in need thereof at a total dose of about 5U-200U as the starting dose and at a frequency of once daily or less, preferably administering the compound of formula (I) and insulin aspart to the subject in need thereof at a frequency of once daily or less and at a total dose of about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 55U, about 60U, about 65U, about 70U, about 75U, about 80U, about 85U, about 90U, about 95U, about 100U, about 110U, about 120U, about 130U, about 140U, about 150U, about 160U, about 170U, about 180U, about 190U, or about 200U.

In some embodiments, after administering the starting dose for 1 day, 2 days, 3 days or 4 days, or after administering for 1, 2, 3, 4, 5, 6, 7, 8, 9, 10, 11, 12, 13, 14, 15 or 16 weeks, the daily dose M is adjusted daily, weekly, or more frequently.

In some embodiments, the daily dose M is adjusted daily, weekly or more frequently according to the following rules:
a) when a fasting blood glucose value of the subject on the day of administration before dosing is 3.9-4.3mmol/L, or when an average of three consecutive prebreakfast fasting capillary blood glucose values of the subject from two or three days before administration is 3.9-4.3mmol/L, the dose M is adjusted to be reduced by about 1U-6U relative to the previous dose, preferably about 1U-4U, preferably about 1U, about 2U, about 3U, or about 4U relative to the previous dose; or
b) when the fasting blood glucose value of the subject on the day of administration before dosing is 4.4-6.1mmol/L, or when the average of three consecutive fasting capillary blood glucose values of the subject before breakfast from two days or three days before administration is 4.4-6.1mmol/L, the dose M is the previous dose; or
c) when the fasting blood glucose value of the subject on the day of administration before dosing is 6.2-7.0mmol/L, or when the average of three consecutive prebreakfast fasting capillary blood glucose values of the subject from two or three days before administration is 6.2-7.0mmol/L, the dose M is adjusted to be increased by about 1U-4U relative to the previous dose, preferably about 1U, about 2U, about 3U or about 4U relative to the previous dose; or
d) when the fasting blood glucose value of the subject on the day of administration before dosing is 7.1-8.0mmol/L, or when the average of three consecutive prebreakfast fasting capillary blood glucose values of the subject from two or three days before administration is 7.1-8.0mmol/L, the dose M is adjusted to be increased by about 2U-6U relative to the previous dose, preferably about 3U, about 4U, about 5U or about 6U relative to the previous dose; or
e) when the fasting blood glucose value of the subject on the day of administration before dosing is 8.1-9.0mmol/L, or when the average of three consecutive prebreakfast fasting capillary blood glucose values of the subject from two or three days before administration is 8.1-9.0mmol/L, the dose M is adjusted to be increased by about 4U-8U relative to the previous dose, preferably about 5U, about 6U, about 7U or about 8U relative to the previous dose; or
f) when the fasting blood glucose value of the subject on the day of administration before dosing is > 9.0mmol/L, or when the average of three consecutive prebreakfast fasting capillary blood glucose values of the subject from two or three days before administration is > 9.0mmol/L, the dose M is adjusted to be increased by about 6U-10U relative to the previous dose, preferably about 7U, about 8U, about 9U or about 10U relative to the previous dose; or
g) when the fasting blood glucose value of the subject on the day of administration before dosing is ≤ 3.0mmol/L, or when the minimum value of three consecutive prebreakfast fasting capillary blood glucose values of the subject from two or three days before administration is ≤ 3.0mmol/L, the dose M is adjusted to be reduced by about 1U-8U relative to the previous dose, preferably by about 2U-6U, preferably by about 3U, about 4U or about 5U relative to the previous dose, or when the previous dose is > 45U, the dose M is adjusted to be reduced by 5% to 15% relative to the previous dose, preferably by 10%; or
h) when the fasting blood glucose value of the subject on the day of administration before dosing is 3.1-3.8mmol/L, or when the minimum value of three consecutive prebreakfast fasting capillary blood glucose values of the subject from two days or three days before administration is 3.1-3.8mmol/L, the dose M is adjusted to be reduced by about 1U-4U relative to the previous dose, preferably by about 1U-3U, preferably by about 1U, about 2U or about 3U relative to the previous dose, or when the previous dose is > 45U, the dose M is adjusted to be reduced by 2% to 10%, preferably by 5% relative to the previous dose.

In some embodiments, the subject is a patient with type II diabetes inadequately controlled by oral antidiabetic drugs; or the subject is a patient with diabetes inadequately controlled by previous use of a basal insulin or premixed insulin; or the subject is a patient with type II diabetes inadequately controlled by previous use of a basal insulin or premixed insulin; or the subject is a patient with type II diabetes inadequately controlled by previous use of a basal insulin once daily or a premixed insulin at least once daily, with or without a mealtime insulin before dinner.In some embodiments, the subject:
has a BMI between 10 kg/m² and 50 kg/m², preferably 18.5-35 kg/m²; and/or
has an HbA1c of 7.5% to 11.0%; and/or
has a fasting venous blood glucose of ≥ 7.0 mmol/L; and/or
has received one or more oral antidiabetic drugs, preferably at most three oral antidiabetic drugs, and the oral antidiabetic drugs include metformin, and further include a combination with or without an oral antidiabetic drug selected from the group consisting of DPP-4 inhibitor, α-glycosidase inhibitor, SGLT2 inhibitor, and glucokinase activator .

In some embodiments, the combination of the compound of formula (I) and insulin aspart, or the pharmaceutical composition comprising the compound of formula (I) and insulin aspart is administered at a fixed or non-fixed time before any meal, preferably before breakfast.

In some embodiments, the combination of the compound of formula (I) and insulin aspart, or the pharmaceutical composition comprising the compound of formula (I) and insulin aspart is administered by subcutaneous injection, preferably by abdominal subcutaneous injection.

In some embodiments, wherein the combination of the compound of formula (I) and insulin aspart, or the pharmaceutical composition comprising the compound of formula (I) and insulin aspart is for long-term administration, preferably for at least 4 weeks, preferably for at least 8 weeks, preferably for at least 16 weeks, preferably for at least 20 weeks, preferably for at least 25 weeks, preferably for at least 32 weeks.

In some embodiments, wherein when the subject is a patient with diabetes inadequately controlled by previous use of a basal insulin or premixed insulin, the method further comprises administering insulin aspart alone to the subject, preferably before dinner daily.

In some embodiments, the method comprises administering the compound of formula (I) and insulin aspart to the subject in need thereof at a total dose of 50%-100% of the previous total daily dose of the basal and/or premixed insulin as a starting dose at a frequency of once daily or less; and/or
further, comprises administering insulin aspart at a total dose of 0%-50% of the previous total daily dose of the basal and/or premixed insulin as the starting dose at a frequency of once daily or twice daily;
wherein, the compound of formula (I) and insulin aspart are administered at a first time point, the insulin aspart is administered at a second time point, and/or the insulin aspart is administered at the second time point and/or a third time point;
preferably, the method comprises administering the compound of formula (I) and the insulin aspart to the subject in need thereof at the first time point with 60%, 70%, 80%, 90% or 100% of the previous total daily dose of the basal and/or premixed insulin as the starting dose at a frequency of once daily or less; and/or
further, comprises administering the insulin aspart once daily or twice daily at the second time point and/or the third time point with 5%, 10%, 15%, 20% or 40% of the previous total daily dose of the basal and/or premixed insulin as the starting dose.

In some embodiments, the method comprises administering the compound of formula (I) and the insulin aspart at a total dose of N and at a frequency of once daily, and/or further administering insulin aspart at a dose of O and at a frequency of once daily or twice daily, after the starting dose is administered for 1 week, wherein the dose N administered each day is the same or different, and/or the dose O administered each day is the same or different; wherein N and O are each independently 0%-100% of the previous total daily dose of the basal and/or premixed insulin, preferably each independently 5%, 10%, 15%, 20%, 25%, 30%, 35%, 40%, 45%, 50%, 55%, 60%, 65%, 70%, 75%, 80%, 85%, 90%, 95% or 100% of the previous total daily dose of the basal and/or premixed insulin.

In some embodiments, the method comprises administering the compound of formula (I) and the insulin aspart at a total dose of N and at a frequency of once daily after the starting dose is administered for 1 week, and/or further administering insulin aspart at a dose of O and at a frequency of once daily or twice daily, wherein the dose N administered each day is the same or different, and/or the dose O administered each day is the same or different; wherein N and O are each independently 0%-100% of the previous total daily dose of the basal and/or premixed insulin, preferably each independently 5%, 10%, 15%, 20%, 25%, 30%, 35%, 40%, 45%, 50%, 55%, 60%, 65%, 70%, 75%, 80%, 85%, 90%, 95% or 100% of the previous total daily dose of the basal and/or premixed insulin;
after administering the starting dose for 1, 2, 3, 4, 5, 6, 7, 8, 9, 10, 11, 12, 13, 14, 15, or 16 weeks, the doses N and O administered each day are adjusted at a frequency of once weekly or more frequently.

In some embodiments, wherein the dose N administered each day is adjusted at a frequency of once weekly or more frequently according to the following rules:
a) when a fasting blood glucose value of the subject on the day of administration before dosing is 3.9-4.3mmol/L, or when an average of three consecutive pre-meal capillary blood glucose values of the subject before administration is 3.9-4.3mmol/L, the dose N is adjusted to be reduced by about 1U-6U relative to the previous dose, preferably about 1U-4U, preferably about 1U, about 2U, about 3U or about 4U relative to the previous dose; or
b) when the fasting blood glucose value of the subject on the day of administration before dosing is 4.4-6.1mmol/L, or when the average of three consecutive pre-meal capillary blood glucose values of the subject before administration is 4.4-6.1mmol/L, the dose N is the previous dose; or
c) when the fasting blood glucose value of the subject on the day of administration before dosing is 6.2-7.0mmol/L, or when the average of three consecutive pre-meal capillary blood glucose values of the subject before administration is 6.2-7.0mmol/L, the dose N is adjusted to be increased by about 1U-4U relative to the previous dose, preferably about 1U, about 2U, about 3U or about 4U relative to the previous dose; or
d) when the fasting blood glucose value of the subject on the day of administration before dosing is 7.1-8.0mmol/L, or when the average of three consecutive pre-meal capillary blood glucose values of the subject before administration is 7.1-8.0mmol/L, the dose N is adjusted to be increased by about 2U-6U relative to the previous dose, preferably about 3U, about 4U, about 5U or about 6U relative to the previous dose; or
e) when the fasting blood glucose value of the subject on the day of administration before dosing is 8.1-9.0mmol/L, or when the average of three consecutive pre-meal capillary blood glucose values of the subject before administration is 8.1-9.0mmol/L, the dose N is adjusted to be increased by about 4U-8U relative to the previous dose, preferably about 5U, about 6U, about 7U or about 8U relative to the previous dose; or
f) when the fasting blood glucose value of the subject on the day of administration before dosing is > 9.0mmol/L, or when the average of three consecutive pre-meal capillary blood glucose values of the subject before administration is > 9.0mmol/L, the dose N is adjusted to be increased by about 6U-10U relative to the previous dose, preferably about 7U, about 8U, about 9U or about 10U relative to the previous dose; or
g) when the fasting blood glucose value of the subject on the day of administration before dosing is ≤ 3.0mmol/L, or when the minimum value of three consecutive pre-meal capillary blood glucose values of the subject before administration is ≤ 3.0mmol/L, the dose N is adjusted to be reduced by about 1U-8U relative to the previous dose, preferably by about 2U-6U, preferably by about 3U, about 4U or about 5U relative to the previous dose, or when the previous dose is > 45U, the dose M is adjusted to be reduced by 5% to 15% relative to the previous dose, preferably by 10%; or
h) when the fasting blood glucose value of the subject on the day of administration before dosing is 3.1-3.8mmol/L, or when the minimum value of three consecutive pre-meal capillary blood glucose values of the subject before administration is 3.1-3.8mmol/L, the dose N is adjusted to be reduced by 1U-4U relative to the previous dose, preferably by 1U-3U, preferably by 1U, 2U or 3U relative to the previous dose, or when the previous dose is > 45U, the dose N is adjusted to be reduced by 2% to 10%, preferably by 5% relative to the previous dose.

In some embodiments, the dose O administered each day is adjusted at a frequency of once weekly or more frequently according to the following rules:
a) when the subject's minimum 2-hour postprandial blood glucose on three consecutive occasions before administration is less than 6.1mmol/L, the dose O is adjusted to be reduced by about 1U-4U relative to the previous dose, preferably by about 1U, about 2U or about 3U; or
b) when the subject's minimum 2-hour postprandial blood glucose on three consecutive occasions before administration is 6.1-6.9mmol/L, the dose O is the previous dose; or
c) when the subject's minimum 2-hour postprandial blood glucose on three consecutive occasions before administration is 7.0- 7.8mmol/L, the dose O is adjusted to be increased by about 0U-4U relative to the previous dose, preferably by about 0U, about 1U, about 2U or about 3U; or
d) when the subject's minimum 2-hour postprandial blood glucose on three consecutive occasions before administration is 7.9- 10.0mmol/L, the dose O is adjusted to be increased by about 0U-4U relative to the previous dose, preferably by about 0U, about 1U, about 2U or about 3U; or
e) when the subject's minimum 2-hour postprandial blood glucose on three consecutive occasions before administration is ≥ 10.0mmol/L, the dose O is adjusted to be increased by about 2U-5U relative to the previous dose, preferably by about 2U, about 3U, about 4U or about 5U.

In some embodiments, the adjustment rules are further adjusted according to the previous dose of the insulin aspart,
when the dose of insulin aspart is less than 15U:
   a) when the subject's minimum 2-hour postprandial blood glucose on three consecutive occasions before administration is less than 6.1mmol/L, the dose O is adjusted to be reduced by about 1U-4U relative to the previous dose, preferably by about 1U, about 2U or about 3U; or
   b) when the subject's minimum 2-hour postprandial blood glucose on three consecutive occasions before administration is 6.1-7.8 mmol/L, the dose O is the previous dose; or
   c) when the subject's minimum 2-hour postprandial blood glucose on three consecutive occasions before administration is 7.9- 10.0mmol/L, the dose O is adjusted to be increased by about 1U-4U relative to the previous dose, preferably by about 1U, about 2U or about 3U; or
   d) when the subject's minimum 2-hour postprandial blood glucose on three consecutive occasions before administration is ≥ 10.0mmol/L, the dose O is adjusted to be increased by about 2U-4U relative to the previous dose, preferably by about 2U, about 3U or about 4U; or
when the dose of insulin aspart is ≥ 15U:
   a) when the subject's minimum 2-hour postprandial blood glucose on three consecutive occasions before administration is less than 6.1mmol/L, the dose O is adjusted to be reduced by about 1U-4U relative to the previous dose, preferably by about 1U, about 2U or about 3U; or
   b) when the subject's minimum 2-hour postprandial blood glucose on three consecutive occasions before administration is 6.1-6.9 mmol/L, the dose O is the previous dose; or
   c) when the subject's minimum 2-hour postprandial blood glucose on three consecutive occasions before administration is 7.0-7.8 mmol/L, the dose O is adjusted to be increased by about 1U-3U relative to the previous dose, preferably by about 1U, about 2U or about 3U; or
   d) when the subject's minimum 2-hour postprandial blood glucose on three consecutive occasions before administration is 7.9-10.0mmol/L, the dose O is adjusted to be increased by about 2U-4U relative to the previous dose, preferably by about 2U, about 3U or about 4U; or
   e) when the subject's minimum 2-hour postprandial blood glucose on three consecutive occasions before administration is ≥ 10.0mmol/L, the dose O is adjusted to be increased by about 3U-5U relative to the previous dose, preferably by about 3U, about 4U or about 5U.

In some embodiments, the first time point, the second time point and the third time point are each independently and optionally selected from before breakfast, before lunch and before dinner; preferably, the first time point is before breakfast, the second time point is before dinner, and the third time point is before lunch.

In some embodiments, wherein the subject:
has a BMI between 10 kg/m² and 50 kg/m², preferably between 18.5 kg/m² and 35 kg/m²; and/or
has an HbA1c of 7.5% to 11.0%; and/or
has a fasting venous blood glucose of ≥ 7.0 mmol/L; and/or
has received one or more oral antidiabetic drugs, preferably has received a treatment of at most three oral antidiabetic drugs combined with one basal insulin or premixed insulin, wherein the dosing frequency of the basal insulin or premixed insulin is no more than twice daily, the daily dose is about 10U-100U, and the duration of the treatment with basal insulin or premixed insulin combined with a stable dose of oral antidiabetic drugs is ≥ 3 months.

In some embodiments, wherein the oral antidiabetic drugs include metformin, which further combines or does not combine with an oral antidiabetic drug selected from the group consisting of DPP4 inhibitor (DPP-4i), α-glycosidase inhibitor, SGLT2 inhibitor, glucokinase activator, sulfonylureas, and glinides.

In some embodiments, the pharmaceutical composition is administered via subcutaneous injection, preferably via abdominal subcutaneous injection.

In some embodiments, the pharmaceutical composition combined with insulin aspart is for a long-term administration, preferably for at least 4 weeks, preferably for at least 8 weeks, preferably for at least 16 weeks, preferably for at least 20 weeks, preferably for at least 25 weeks, preferably for at least 32 weeks.

In some embodiments, the subject further receives daily oral administration of metformin.

In some embodiments, in the pharmaceutical composition comprising the compound of formula (I) and insulin aspart, the content of the compound of formula (I) is about 20-200U/ml, preferably about 30-100U/ml, preferably about 30U/ml, about 40U/ml, about 50U/ml, about 60U/ml, about 70U/ml, about 80U/ml, about 90U/ml, about 100U/ml, preferably about 30U/ml, about 40U/ml, about 50U/ml, about 60U/ml, or about 70U/ml.

In some embodiments, the pharmaceutical composition comprising the compound of formula (I) and insulin aspart further comprises 10-25mg/ml glycerol, 1.5-6mg/ml phenol, 0.5-3mg/ml *m*-cresol, 5-50 µg/ml zinc ions and 0.5-2.0mg/ml sodium chloride, and the pH of the composition is 7.0-8.0;
preferably, the pharmaceutical composition comprising the compound of formula (I) and insulin aspart further comprises 10-25mg/ml glycerol, 1.5-3.5mg/ml phenol, 0.5-2.0mg/ml *m*-cresol, 5-50µg/ml zinc ions and 0.5-2.0mg/ml sodium chloride, and the pH of the composition is 7.0-8.0;
preferably, the pharmaceutical composition comprising the compound of formula (I) and insulin aspart further comprises 17mg/ml glycerol, 2.82mg/ml phenol, 1.08mg/ml *m*-cresol, 15-40 µg/ml zinc ions and 1.17mg/ml sodium chloride, and the pH of the composition is 7.2-7.8;
preferably, the pharmaceutical composition comprises 0.18mM of the compound of formula (I), 0.18mM insulin aspart, 17mg/ml glycerol, 2.82mg/ml phenol, 1.08mg/ml *m*-cresol, 18.75 µg/ml zinc ions and 1.17mg/ml sodium chloride, and the pH of the composition is 7.2-7.8;
or the pharmaceutical composition comprises 0.42mM of the compound of formula (I), 0.18mM insulin aspart, 17mg/ml glycerol, 2.82mg/ml phenol, 1.08mg/ml *m*-cresol, 35.9 µg/ml zinc ions and 1.17mg/ml sodium chloride, and the pH of the composition is 7.2-7.8;
or the pharmaceutical composition comprises 0.3mM of the compound of formula (I), 0.3mM insulin aspart, 17mg/ml glycerol, 2.82mg/ml phenol, 1.08mg/ml *m*-cresol, 31.25 µg/ml zinc ions and 1.17mg/ml sodium chloride, and the pH of the composition is 7.2-7.8.

Another aspect of the disclosure provides a method for treating metabolic syndrome, wherein the method comprises administering to a subject in need thereof a compound of formula (I), or a pharmaceutically acceptable salt, amide or ester thereof, 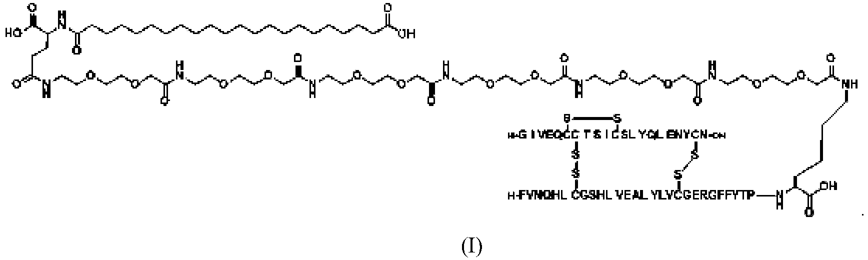
preferably, the metabolic syndrome is diabetes mellitus; preferably, the diabetes mellitus is type I diabetes or type II diabetes;
preferably, the subject is a patient with diabetes inadequately controlled by oral antidiabetic drugs, preferably a patient with type II diabetes;
preferably, the subject is a patient with diabetes inadequately controlled by previous use of a basal insulin or premixed insulin, preferably a patient with type II diabetes.

In some embodiments, a single dose of the compound of formula (I) is calculated according to the body weight of the subject, and the single dose is 0.2-30nmol/kg, preferably 0.45nmol/kg, 0.9nmol/kg, 1.8 nmol/kg, 3.6nmol/kg, 7.2nmol/kg, 14.4nmol/kg or 28.8nmol/kg.

In some embodiments, the single dose of the compound of formula (I) is calculated according to the body weight of the subject, and the single dose is 0.03-5U/kg, preferably about 0.075U/kg, about 0.15U/kg, about 0.3U/kg, about 0.6U/kg, about 1.2U/kg, about 2.4U/kg or about 4.8U/kg.

In some embodiments, the subject achieves a reduction in glycated hemoglobin by at least 0.1% after administering the combination or the pharmaceutical composition of the compound of formula (I) and insulin aspart relative to that before administration, preferably by at least 0.2%, preferably by at least 0.3%, preferably by at least 0.4%, preferably by at least 0.5%, preferably by 0.5%-30%, preferably by 0.6%-25%, preferably by 0.62%, 0.76%, 0.9%, 1%, 2%, 3%, 4%, 5%, 6%, 6.5%, 7%, 8%, 9%, 10%, 11%, 12%, 13%, 13.5%, 14%, 15%, 16%, 17%, 18%, 18.6%, 19%, or 20%; preferably, the subject achieves a reduction in glycated hemoglobin by at least 0.1% after administering the combination or the pharmaceutical composition of the compound of formula (I) and insulin aspart for 16 weeks, 20 weeks, 25 weeks, 30 weeks, or 35 weeks, preferably by at least 0.2%, preferably by at least 0.3%, preferably by at least 0.4%, preferably by at least 0.5%, preferably by 0.5%-30%, preferably by 0.6%-25%, preferably by 0.62%, 0.76%, 0.9%, 1%, 2%, 3%, 4%, 5%, 6.5%, 7%, 8%, 9%, 10%, 11%, 12%, 13%, 13.5%, 14%, 15%, 16%, 17%, 18%, 18.6%, 19%, or 20%.

In some embodiments, the difference between the area under the time curve of glucose infusion rate (GIR) within 0-24 hours and the area under the time curve of glucose infusion rate (GIR) within 24-48 hours after administration of the compound having the structure of formula (I) is less than 20hmg/kg/min, preferably, the difference is 2-15 hmg/kg/min, more preferably, the difference is 2-10 hmg/kg/min.

In some embodiments, the difference between the GIRmax of glucose infusion rate (GIR) within 0-24 hours and GIRmax within 24-48 hours after administration of the compound having the structure of formula (I) is less than 1.5mg/kg/min, preferably, the difference is 0-1.2hmg/kg/min, more preferably, the difference is 0-0.6 hmg/kg/min.

In some embodiments, after administration of the compound having the structure of formula (I) for 2 to 10 consecutive days, the compound having the structure of formula (I) exhibits a smooth PK profile, wherein the difference between Ctrough and Cmax of the compound having the structure of formula (I) at steady state is less than 8ng/mL, preferably less than 7ng/mL, preferably less than 6ng/mL.

In some embodiments, the administration of the compound having the structure of formula (I) exhibits a more prolonged and stable hypoglycemic effect than insulin degludec.

Another aspect of the disclosure provides a kit, comprising:
a compound of formula (I), or a pharmaceutically acceptable salt thereof, 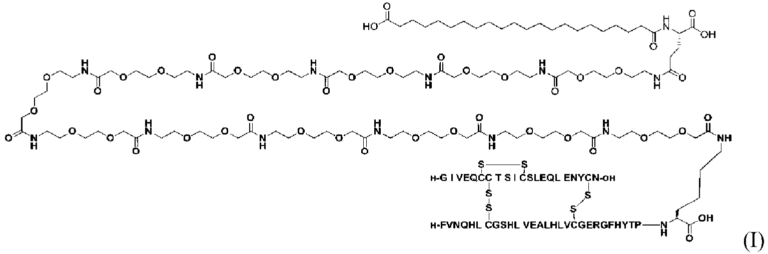
a packaging material, and
a label or package insert contained in the packaging material, the label or package insert providing instructions for treating a subject with the compound of formula (I), or the pharmaceutically acceptable salt thereof, according the method described above.

### BRIEF DESCRIPTION OF THE DRAWINGS

FIG. 1 illustrates a graph showing the change from baseline in fasting blood glucose (Mean±SE) over time in Example 1;
FIG. 2 illustrates a graph showing the change from baseline in fasting blood glucose (Mean±SE) over time in Example 1.

### Definitions and Explanations

Unless otherwise specified, the following terms and phrases used herein are intended to have the meanings set forth below. A particular term or phrase should not be considered ambiguous or unclear in the absence of a specific definition, but should be understood in its ordinary sense. When trade names appear in this document, they are intended to refer to the corresponding products or their active ingredients.

As used herein and unless otherwise specified, the terms "comprise," "include," and "have," including their grammatical equivalents, are generally to be understood as open-ended and non-limiting, for example, not excluding other unlisted elements or steps.

In this application, when referring to specifically listed values or ranges of values, the term "about"as used herein generally means within 20%, preferably within 10%, and more preferably within 5% of the given value or range.

The term "basic insulin" refers to insulin having a longer duration of action than normal or human insulin.

The term "insulin" includes naturally occurring insulin, such as human insulin, as well as its insulin analogs and insulin derivatives. The present invention also protects analogs and derivative forms of acylated insulin equivalent to the compound shown in Formula (I).

The term "insulin aspart" refers to insulin produced by recombinant DNA technology, wherein the amino acid at position B28 (i.e., the 28th amino acid in the human insulin B chain) of human insulin (which is proline) is replaced with aspartic acid.

The term "insulin analog" comprises peptides having a molecular structure derived from a naturally occurring insulin (e.g., human insulin) through the deletion and/or substitution (replacement) of one or more amino acid residues present in natural insulin and/or the addition of one or more amino acid residues. The added and/or substituted amino acid residues may be encodable amino acid residues, other naturally occurring amino acid residues, or purely synthetic amino acid residues. Preferably, the added and/or substituted amino acid residues are encodable amino acid residues.

The term "insulin derivative" herein refers to naturally occurring insulin or insulin analogs that have been chemically modified, such modifications may include, for example, introducing side chains at one or more positions on the insulin backbone, oxidizing or reducing groups on amino acid residues of insulin, or converting free carboxyl groups into ester groups or acylating free amino or hydroxyl groups. The acylated insulin of the present invention falls within the category of insulin derivatives.

The term "insulin parent" refers to the insulin portion of an insulin derivative or acylated insulin (also referred to herein as parent insulin), for example, in the present invention, it refers to the portion of an insulin derivative or acylated insulin that has no attached side chains or acyl groups. The insulin parent may be naturally occurring insulin, such as human insulin or porcine insulin. On the other hand, the parent insulin may be an insulin analog.

The term "amino acid residue" herein includes amino acids from which a hydrogen atom has been removed from the amino group and/or a hydroxyl group has been removed from the carboxyl group and/or a hydrogen atom has been removed from the thiol group. Imprecisely, amino acid residues may also be referred to as amino acids.

Unless otherwise specified, the amino acids referred to herein are all L-amino acids.

The term "hypoglycemic event" is determined based on the lowest value of the subject's fasting capillary blood glucose measured before breakfast for three consecutive times starting from two days prior to administration (including the day of the visit). If the lowest value of the subject's fasting capillary blood glucose measured before breakfast for three consecutive times starting from two days prior to administration (including the day of the visit) is ≥3.9 mmol/L, the subject is determined as not having experienced a hypoglycemic event. Conversely, if said value is <3.9 mmol/L, the subject is determined as having experienced a hypoglycemic event.

The term "treatment" includes therapeutic treatment, prophylactic treatment, and application in reducing the subject's risk of developing a disease or other risk factors. Treatment includes, but is not limited to, complete cure of a disease, as well as alleviation of symptoms or reduction of underlying risks.

The term "metabolic syndrome" as used herein is a recognized clinical term used to describe a condition that includes a combination of the following: type II diabetes, impaired glucose tolerance, insulin resistance, hypertension, obesity, increased abdominal circumference, hypertriglyceridemia, low HDL, hyperuricemia, hypercoagulable state, and/or microalbuminuria.

The term "type II diabetes" (referred to as "type 2 diabetes," previously known as "non-insulin-dependent diabetes" or "adult-onset diabetes") accounts for 90-95% of all diabetes cases and encompasses individuals who have insulin resistance and usually have relative (rather than absolute) insulin deficiency.

"Anti-diabetes treatment" for type 2 diabetes includes:
Metformin: Generally, metformin is the first prescribed pharmaceutical for type 2 diabetes. It works by increasing the sensitivity of body tissues to insulin, allowing the body to use insulin more effectively. Metformin also reduces glucose production in the liver. Metformin alone may not lower blood glucose.
Alpha-glucosidase inhibitors: Alpha-glucosidase inhibitors delay carbohydrate absorption by inhibiting alpha-glucosidase at the brush border of the small intestinal mucosa, thereby reducing postprandial hyperglycemia. Their main features include stable glucose-lowering effects, high safety, and the ability to reduce the incidence of cardiovascular complications. They are among the few oral hypoglycemic drugs that can intervene in impaired glucose tolerance. Commonly used alpha-glucosidase inhibitors are primarily acarbose and voglibose.
Dipeptidyl peptidase-4 (DPP-4) inhibitors: These pharmaceuticals also lower blood glucose levels. They do not cause weight gain. Examples of these pharmaceuticals are sitagliptin, saxagliptin, vildagliptin, and linagliptin.
Sulfonylureas: These pharmaceuticals help the body secrete more insulin. Examples of this class include glyburide, glipizide, and glimepiride. Possible side effects include hypoglycemia and weight gain.
Thiazolidinediones: Like metformin, these pharmaceuticals make the body's tissues more sensitive to insulin. These pharmaceuticals are associated with weight gain and other more serious side effects, such as an increased risk of heart failure and fractures. Due to these risks, these pharmaceuticals are typically not the first-line treatment. Pioglitazone is an example of a thiazolidinedione.
SGLT2 inhibitors: These are the newest class of diabetes pharmaceuticals on the market. They work by preventing the kidneys from reabsorbing sugar back into the blood. Instead, glucose is excreted in the urine. Examples include canagliflozin and dapagliflozin.

In this application, the term "compound" refers to a molecular entity; therefore, a "compound" may possess different structural elements in addition to the minimal elements defined for each compound or group of compounds. The term "compound" is intended to comprise its pharmaceutically relevant forms; that is, the present invention relates to the compounds defined herein or their pharmaceutically acceptable salts, amides, or esters.

The term "pharmaceutically acceptable" refers to those compounds, compositions, and/or formulations that, within the scope of sound medical judgment, are suitable for use in contact with human and animal tissues without excessive toxicity, irritation, allergic reactions, or other problems or complications, commensurate with a reasonable benefit/risk ratio.

The salts may be basic salts, acidic salts, or neither (i.e., neutral salts). In water, basic salts produce hydroxide ions, while acidic salts produce hydrated hydrogen ions.

Salts of the derivatives of the present invention may be formed by adding cations or anions that react with anionic or cationic groups, respectively. These groups may be located in the peptide moiety and/or in the side chains of the derivatives of the present invention.

Non-limiting examples of anionic groups in the derivatives of the present invention include free carboxyl groups in the side chain (if any) and in the peptide moiety. The peptide moiety typically contains a free carboxyl group at the C-terminus and may also contain free carboxyl groups at internal acidic amino acid residues such as Asp and Glu.

Non-limiting examples of cationic groups in the peptide moiety include the free amino group at the N-terminus (if any), as well as any free amino groups of internal basic amino acid residues such as His, Arg, and Lys.

Esters of the derivatives of the present invention may be formed, for example, by the reaction of a free carboxyl group with an alcohol or phenol, a reaction that results in the substitution of at least one hydroxyl group with an alkoxy or aryloxy group.

Ester formation may involve a free carboxyl group at the C-terminus of the peptide and/or any free carboxyl group in the side chains.

Amides of the derivatives of the present invention may be formed, for example, by reacting a free carboxyl group in an activated form with an amine or a substituted amine, or by reacting a free or substituted amino group with a carboxylic acid in an activated form.

Amide formation may involve a free carboxyl group at the C-terminus of the peptide, any free carboxyl group in the side chain, a free amino group at the N-terminus of the peptide, and/or any free or substituted amino group in the peptide and/or the side chain.

In a specific embodiment, the peptide or derivative is in the form of a pharmaceutically acceptable salt. In another specific embodiment, the derivative is in the form of a pharmaceutically acceptable amide, preferably comprising an amide group at the C-terminus of the peptide. In a further specific embodiment, the peptide or derivative is in the form of a pharmaceutically acceptable ester.

In this application, HbA1c refers to glycated hemoglobin. Glycated hemoglobin control targets follow a patient-centered individualized principle, and currently, most diabetes guidelines recommend an HbA1c target of <7.0% for general adult patients with T2DM.

### II. Examples

The embodiments of the present invention will be described in detail below with reference to the examples. However, it will be understood by those skilled in the art that the following examples are only intended to illustrate the present invention and should not be construed as limiting the scope of the present invention. In the examples, where specific conditions are not indicated, the procedures are carried out under conventional conditions or conditions recommended by the manufacturer. Reagents or instruments whose manufacturers are not specified are all conventional products that are commercially available.

### Preparation Example

The injection formulation of Pharmaceutical Composition 1 used in the following examples is as follows: 0.18 mM Compound (I), 0.18 mM insulin aspart, 17 mg/ml glycerol, 2.82 mg/ml phenol, 1.08 mg/ml m-cresol, 18.75 µg/ml zinc ions, and 1.17 mg/ml sodium chloride. The pH of this composition is 7.2-7.8.

The injection formulation of Pharmaceutical Composition 2 is as follows: 0.42 mM Compound (I), 0.18 mM insulin aspart, 17 mg/ml glycerol, 2.82 mg/ml phenol, 1.08 mg/ml m-cresol, 35.9 µg/ml zinc ions, and 1.17 mg/ml sodium chloride. The pH of this composition is 7.2-7.8. Compound (I) can be prepared, for example, with reference to the preparation method described in WO2021136302.

Compound (I): B29K(*N(ε)*-docosanedioyl-γGlu-6xOEG), desB30 human insulin (SEQ ID NO: 1 and SEQ ID NO: 2, representing the A-chain and B-chain, respectively). 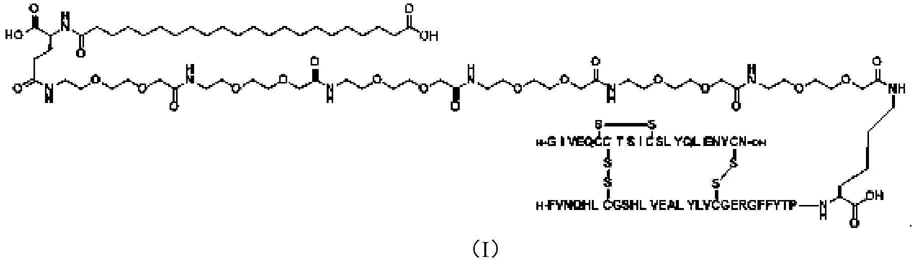
SEQ ID NO.1:
   DesB30 human insulin A-chain:
   Gly Ile Val Glu Gln Cys Cys Thr Ser Ile Cys Ser Leu Tyr Gln Leu Glu Asn Tyr Cys Asn
SEQ ID NO.2:
   DesB30 human insulin B-chain:
   Phe Val Asn Gln His Leu Cys Gly Ser His Leu Val Glu Ala Leu Tyr Leu Val Cys Gly Glu Arg Gly Phe Phe Tyr Thr Pro Lys

### Example 1: Clinical Trial of Pharmaceutical Composition 1 Comprising Compound (I) and Insulin Aspart in Adult Patients with Type 2 Diabetes Inadequately controlled on Oral Antidiabetic Drugs

### Trial Protocol

This clinical trial is a randomized, double-blind, active-controlled trial designed to evaluate the efficacy, safety, and tolerability of Pharmaceutical Composition 1 in adult patients with type 2 diabetes inadequately controlled on oral antidiabetic drugs. The medications used are shown in Table 1.

**Table 1: Formulation Names, Specifications, and Administration Methods of Investigational Drug and Active Control Drug**

| | Investigational Drug | Active Control Drug |
|---|---|---|
| Formulation Names | Pharmaceutical Composition Comprising Compound of Formula (I) and Insulin Aspart | Insulin Degludec and Insulin Aspart Injection (Ryzodeg^{®}) |
| Specifications | 3 mL: 180 U( Compound (I): 30 U/mL; Insulin Aspart: 30 U/mL) | 3 mL: 300 U |
| Manufacturer | Gan & Lee Pharmaceuticals | Novo Nordisk A/S |
| Administration Methods | Subcutaneous injection (at a site at least approximately two finger-widths away from the periumbilical area of the abdomen) | Subcutaneous injection (at a site at least approximately two finger-widths away from the periumbilical area of the abdomen) |

| | | |
|---|---|---|
| Note: Before use, the insulin injections should be equilibrated to room temperature (recommended for approximately 30 minutes). | | |

### Study Methods

The study population enrolled in the trial consists of adult patients with type 2 diabetes inadequately controlled on oral antidiabetic drugs. A multicenter, randomized, open-label, parallel-controlled trial design is employed, aiming to compare the efficacy, safety, and tolerability of once-daily Compound of Formula (I) injection versus Insulin Degludec/Insulin Aspart injection in patients with type 2 diabetes inadequately controlled on oral antidiabetic drugs.

The study included a screening period of up to 17 days. Eligible subjects after screening were stratified based on their glycated hemoglobin (HbA1c) levels (stratification factors: HbA1c ≥ 8.5%, HbA1c < 8.5%) and randomly assigned in a 1:1 ratio to either the Pharmaceutical Composition 1 injection group (Group A1) or the Insulin Degludec/Insulin Aspart injection group (Group A2). The study drug is administered by abdominal subcutaneous injection.

During the treatment period, investigators adjusted the dose of Pharmaceutical Composition 1 injection or Insulin Degludec/Insulin Aspart injection weekly based on the subject's pre-breakfast fasting capillary blood glucose results until the fasting blood glucose control target was achieved [i.e., the average of three consecutive pre-breakfast fasting capillary blood glucose measurements is between 4.4 and 6.1 mmol/L (inclusive)]. A 2-week safety follow-up period followed the end of the treatment period.

This study selected the marketed Insulin Degludec/Insulin Aspart injection (Insulin Degludec/Insulin Aspart injection) as the active control drug. This is intended to protect subjects from ineffective placebo exposure while exploring the difference between Pharmaceutical Composition 1 injection and Insulin Degludec/Insulin Aspart injection in lowering blood glucose in adult patients with type 2 diabetes.

### Subject Inclusion and Exclusion Criteria

The inclusion and exclusion criteria for subjects are shown in Table 2.

**Table 2: Subject Inclusion and Exclusion Criteria (Eligible subjects must meet all inclusion criteria; subjects who meet one or more of the exclusion criteria will be excluded)**

| | Limit |
|---|---|
| Inclusion Criteria | 1. Signed the Informed Consent Form (ICF) before the trial, have a full understanding of the trial content, procedures, and possible adverse reactions, and be able to comply with the prohibitions and restrictions specified in this protocol. |
| | 2. Aged between 18 and 75 years (inclusive) at the time of informed consent, male or female. |
| | 3. Have been receiving up to 3 oral antidiabetic drugs prior to screening, with the doses of oral antidiabetic drugs remaining stable for ≥ 3 months before randomization. The oral antidiabetic drugs must include metformin (requiring a metformin dose of ≥ 1.5 g or the maximum tolerated dose before screening), and any concomitant oral antidiabetic drugs are limited to DPP-4 inhibitors, alpha-glucosidase inhibitors, SGLT2 inhibitors, and glucokinase activators. |
| | 4. No prior use of insulin therapy, except for previous short-term insulin therapy (up to 14 days in total) and insulin therapy for gestational diabetes before screening. |
| | 5. At screening, female subjects of childbearing potential must have a negative high-sensitivity serum beta-human chorionic gonadotropin (β-hCG) pregnancy test result. |
| | 6. Male or female subjects must agree to use reliable contraception for themselves and their partners within 3 months after the last dose (the contraceptive measures used should comply with local regulations regarding contraceptive methods for subjects participating in clinical studies, see Appendix 2 for details), and must not donate sperm or eggs (ova, oocytes) for the purpose of assisted reproduction. |
| | 7. BMI between 18.5 and 35 kg/m² (inclusive) at screening. |
| | 8. Diagnosis of type 2 diabetes mellitus for ≥ 6 months, according to the 1999 World Health |
| | Organization (WHO) diagnostic and classification criteria for diabetes mellitus, and the supplemental diagnostic criteria based on the WHO recommendation on the use of HbA1c in diagnosis (2011). |
| | 9. Mean pre-breakfast fasting capillary blood glucose ≤ 13.9 mmol/L on 3 consecutive days before randomization (including the day of randomization), with 7.0% ≤ HbA1c ≤ 11.0% and fasting venous plasma glucose ≥ 7.0 mmol/L at screening. |
| Exclusion Criteria | 1. History of drug abuse within 1 year before screening, or history of alcoholism within 6 months before screening. |
| | 2. Contraindications to metformin and/or DPP-4i (for the population using metformin in combination with DPP-4i) and/or SGLT2i (for the population using metformin in combination with SGLT2i) and/or alpha-glucosidase inhibitors (for the population using metformin in combination with alpha-glucosidase inhibitors) and/or glucokinase activators (for the population using metformin in combination with glucokinase activators) present before screening. |
| | 3. Pregnant or breastfeeding women. |
| | 4. A definite diagnosis of any malignant tumor before screening, or a documented history of malignant tumor. |
| | 5. Bleeding tendency (including but not limited to non-traumatic hemorrhage) within 6 months before screening, or a definite diagnosis of a disease that increases bleeding tendency. |
| | 6. Confirmed or suspected diagnosis of type 1 diabetes mellitus, gestational diabetes mellitus, or other specific types of diabetes (monogenic diabetes syndromes, cystic fibrosis, pancreatitis, drug- or chemical-induced diabetes, etc.) before screening. |
| | 7. Within 6 months before screening, diabetic ketoacidosis, diabetic lactic acidosis, or hyperosmolar non-ketotic diabetic coma, or unstable or requiring treatment for proliferative retinopathy or maculopathy, diabetic neuropathy, intermittent claudication, or diabetic foot, which, in the investigator's judgment, may affect the clinical study. |
| | 8. Severe hypoglycemia (Grade 3 hypoglycemia) event within 3 months before screening, or recurrent symptoms related to hypoglycemia (once a week or more) within 1 month before randomization, judged by the investigator as unsuitable for enrollment. |
| | 9. Any unstable or further treatment-requiring endocrine system disease related to blood glucose (such as hyperthyroidism, acromegaly, Cushing's syndrome) or immune system disease, judged by the investigator as unsuitable for participation in the study. |
| | 10. History of acute or chronic pancreatitis, history of symptomatic acute or chronic gallbladder disease, history of pancreatic injury, or other high-risk factors that may lead to pancreatitis before screening. |
| | 11. Unstable controlled hypertension (systolic blood pressure ≥ 180 mmHg and/or diastolic blood pressure ≥ 100 mmHg) at screening. |
| | 12. History of invasive cardiovascular surgery before screening or planned cardiovascular surgery |
| | during the trial period. Or history of acute heart failure within 6 months before screening, or hospitalization for coronary heart disease, myocardial infarction, unstable angina, stroke, etc. within 6 months before screening. Or indication for pacemaker implantation at screening, or second- or third-degree atrioventricular block, long QT syndrome, or QTc interval > 450 ms on 12-ECG without a pacemaker at screening. History of chronic heart failure classified as New York Heart Association Class III or IV at screening. Or other types of heart disease present at screening that the investigator considers make the subject ineligible for the trial or may affect the judgment of trial results. |
| | 13. History of allergy to ≥ 2 drugs, or known allergy to the study drug or its excipients (glycerol, phenol, m-cresol, zinc ions, sodium chloride, disodium hydrogen phosphate), hypersensitivity, or intolerance. |
| | 14. History of severe infection or major surgery within 3 months before screening that the investigator judges would affect the conduct of this trial or interfere with the investigator's interpretation of the results, or other systemic diseases present at screening that the investigator judges would affect the conduct of this trial or interfere with the investigator's interpretation of the results (excluding confirmed complications of type 2 diabetes), such as respiratory system diseases, digestive system diseases, genitourinary system diseases, hematological system diseases (e.g., aplastic anemia, myelodysplastic syndrome, thalassemia, sickle cell anemia, hemolytic anemia, etc.), or any disease causing hemolysis or erythrocyte instability (e.g., malaria, Henoch-Schönlein purpura, etc.), musculoskeletal system diseases, immune system diseases, psychiatric or neurological diseases, etc. |
| | 15. Donation of blood or blood products (exceeding 100 mL) or significant blood loss (exceeding 450 mL) within 2 months before screening, or intention to donate blood or blood products during the study period. |
| | 16. Presence of the following laboratory abnormalities at screening: |
| | 1) Hematological abnormalities: Hemoglobin < 110 g/L (male) or < 100 g/L (female), or white blood cell count < 3 × 10⁹/L, or neutrophil count < 2×10⁹/L, or platelet count < 100×10⁹/L, or international normalized ratio (INR) > 1.5. |
| | 2) Serum alanine aminotransferase (ALT) and/or aspartate aminotransferase (AST) > 2 times the upper limit of normal (ULN). Or total bilirubin > 1.5 times the upper limit of normal (ULN). Or elevated serum amylase and/or serum lipase ≥ 1.5 times the upper limit of normal (ULN) or abnormality considered clinically significant. Or fasting triglyceride (TG) > 5.7 mmol/L. Or eGFR < 45 mL/min/1.73 m² [using the Chronic Kidney Disease Epidemiology Collaboration (CKD-EPI) formula, see Appendix 3 for details]. |
| | 3) TSH value outside the normal range ± 10%. |
| | 4) Positive serological test result for human immunodeficiency virus (HIV) antibody, or positive test result for Treponema pallidum, or positive test for hepatitis B virus (HBV) surface antigen with |
| | positive anti-hepatitis B core antibody IgM (IgM anti-HBc) (to exclude patients with acute hepatitis B infection), or positive serological reaction for hepatitis C virus (HCV) antibody [except if the subject had two negative HCV RNA test results 6 months apart before screening, and a third HCV RNA test obtained at screening is also negative]. |
| | 17. Presence of other clinically significant abnormalities in physical examination, vital signs, laboratory tests, imaging examinations, 12-ECG, or other auxiliary examinations at screening that the investigator considers would make the subject ineligible for the trial or could affect the judgment of trial results. |
| | 18. Participation (having received investigational product) in a clinical study of another investigational drug within a period before randomization (1 month or 5 half-lives of the other investigational drug, whichever is longer), or currently participating in another clinical study, or intending to participate in another clinical study before completing all scheduled assessments in this clinical study. |
| | 19. Investigator's judgment that the subject is unable to comply with the requirements of this protocol, such as unwillingness to adhere to relevant lifestyle restrictions during the trial, inability to take medication and eat meals on time as required by the protocol, inability to perform and record SMBG in a timely manner, inability to cooperate with CGM examinations, or any other condition the investigator considers unsuitable for participation in this study. |
| | 20. Treatment with meglitinides, sulfonylureas, thiazolidinediones (TZDs), GLP-1 receptor agonists, GLP-1R/GCGR agonists, GIPR/GLP-1R agonists, GIPR/GLP-1R/GCGR agonists, or traditional Chinese medicines or health products whose indications in the package insert suggest a glucose-lowering effect within 3 months before screening. |
| | 21. Use of medications indicated for obesity within 3 months before screening or anticipated initiation or change of concomitant medications known to affect body weight or glucose metabolism during the trial period. |
| | 22. Investigator has a clear reason to believe that participation in this study would harm the subject's rights and interests. |
| | 23. Subject is an employee of the investigator or the study center, or a family member of an employee or the investigator. |

If the subject meets the inclusion criteria and does not meet any exclusion criteria, they will be assigned to groups according to the randomization principle and receive 16 weeks of treatment. The insulin administration methods are shown in Table 3. During the treatment period, the investigator will adjust the insulin dose weekly based on the subject's self-monitored pre-breakfast fasting capillary blood glucose results (three consecutive measurements, including the visit day, starting from two days before the dose administration). The dose adjustment rules are shown in Table 4 and Table 5, with the adjustment target being a mean pre-breakfast fasting capillary blood glucose value between 4.4 and 6.1 mmol/L (inclusive) over three consecutive days.

**Table 3: Insulin Administration Methods During the Treatment Period**

| Group | Number of Subjects | Medication | Administration Method | Starting Dose | Administration Time |
|---|---|---|---|---|---|
| A1 | 31 | Pharmaceutical Composition containing Compound of Formula (I) and Insulin Aspart | Subcutaneous injection in the abdomen | 10 U | Once daily before breakfast |
| A2 | 31 | Insulin Degludec/Insulin Aspart Injection | Subcutaneous injection in the abdomen | 10 U | Once daily before the same meal |

**Table 4: Weekly Insulin Dose Adjustment Rules (Absence of Hypoglycemic Events*)**

| Average of 3 Consecutive Pre-Breakfast Fasting Capillary Blood Glucose Starting from 2 Days Before Dose Administration (mmol/L) | Insulin Dose Adjustment (U/Week) |
|---|---|
| 3.9-4.3 | -2 |
| 4.4-6.1 | No adjustment |
| 6.2-7.0 | +2 |
| 7.1-8.0 | +4 |
| 8.1-9.0 | +6 |
| >9.0 | + 8 |

| | |
|---|---|
| Note: *Refers to instances where the minimum value of three consecutive pre-breakfast fasting capillary blood glucose measurements (including the visit day) starting from two days before the dose administration is ≥ 3.9 mmol/L. If one or more pre-breakfast fasting capillary blood glucose values are missing, dose adjustment should be based on the remaining SMBG values. If the subject's fasting blood glucose fails to reach the predetermined treatment target, the investigator may, depending on the subject's condition, individualize the insulin dose adjustment rules after communication with the sponsor. | |

**Table 5: Weekly Insulin Dose Adjustment Rules (In the Presence of Hypoglycemic Events*)**

| Minimum of 3 Consecutive Pre-Breakfast Fasting Capillary Blood Glucose Starting from 2 Days Before Dose Administration (mmol/L) | Insulin Dose Adjustment (U/Week) |
|---|---|
| ≤3.0 | -4 (If dose > 45 U, reduce by 10%) |
| 3.1-3.8 | -2 (If dose > 45 U, reduce by 5%) |

| | |
|---|---|
| Note: *Refers to any instance of pre-breakfast fasting capillary blood glucose < 3.9 mmol/L among the three consecutive measurements (including the visit day) starting from two days before the dose administration. If a subject experiences pre-breakfast fasting capillary blood glucose < 3.9 mmol/L on days 1-4 after the dose administration at the weekly visit, or experiences random blood glucose < 3.9 mmol/L after any dose administration, dose adjustment according to Table 3 is also required. This applies unless there is a clear explanation or precipitating factor for the minimum value, such as a missed meal, etc. | |

If the administration of the Pharmaceutical Composition 1 injection is missed at breakfast in Group A1, it is recommended to supplement the missed dose at lunch. If the next main meal is dinner, it is recommended to supplement half of the missed dose. The regular dosing regimen should be resumed the next day. If the administration of the Insulin Degludec/Insulin Aspart injection is missed in Group A2, it is recommended to supplement the missed dose at the next main meal of the same day. The regular dosing regimen should be resumed the next day.

In addition to the treatment according to Tables 3-5, subjects will concomitantly take metformin and/or other oral medications. The frequency and dose of metformin and/or other oral medications taken by the subjects will be the same as their regimen before this trial.

If a subject's fasting blood glucose fails to reach the predetermined treatment target or if hypoglycemic events occur, the investigator may, depending on the subject's condition, individualize the insulin dose adjustment rules after communication with the sponsor.

### Analysis Methods

Intention-to-Treat (ITT) Analysis Set: Includes all subjects randomized into the study.

Full Analysis Set (FAS): Based on the ITT principle, includes all subjects who were randomized and had at least one record of study drug administration.

Per Protocol Set (PPS): A subset of the FAS, including all subjects with good compliance during the study period, complete primary efficacy endpoint data, no major protocol deviations affecting the primary efficacy endpoint, and who must have received insulin (study drug) treatment for at least 12 weeks.

### Study Results

### Subject Disposition

The 62 successfully screened subjects were randomly assigned in a 1:1 ratio to the Pharmaceutical Composition 1 injection group (Group A1) and the Insulin Degludec/Insulin Aspart injection group (Group A2), all of whom received the study drug (31 subjects in Group A1, 31 subjects in Group A2). Among these, one subject (Group A1) voluntarily withdrew consent early from the trial, while the remaining 61 subjects (98.4%) completed the trial according to the protocol.

All 62 subjects (100%) were included in the ITT, FAS, PDAS, and SS. Due to poor compliance, missing primary efficacy endpoint data, or other factors affecting the primary efficacy evaluation, three subjects in Group A1 were not included in the PPS. The remaining 59 subjects (95.2%) were included in the PPS. All 31 subjects in Group A1 were included in the PKAS and IS.

### Demographic Distribution and Baseline Characteristics

Baseline demographics and diabetes-related baseline characteristics were generally balanced between subjects in Group A1 and Group A2.

### Efficacy Results

### (1) Analysis of Glycated Hemoglobin Reduction

Based on the FAS, an ANCOVA model (with MI imputation) was used to compare the changes from baseline in HbA1c after 16 weeks of treatment between the groups. After 16 weeks of treatment, the mean (SD) HbA1c values for subjects in Group A1 and Group A2 were 6.83 (0.701)% and 7.06 (0.728)%, respectively. The least squares mean (standard error) changes from baseline were -1.50 (0.127)% and -1.32 (0.125)%, respectively. The results demonstrate that after 16 weeks of treatment, the reduction in HbA1c in Group A1 (Pharmaceutical Composition 1 injection group) was greater than that in Group A2 (Insulin Degludec/Insulin Aspart injection group).

Based on the FAS population, a summary of the HbA1c analysis for subjects during the treatment period is shown in Table 6 below.

**Table 6: HbA1c (%) Analysis**

| **Statistical Parameter** | Pharmaceutical Composition 1 Injection (N = 31) | Insulin Degludec/Insulin Aspart Injection (N = 31) |
|---|---|---|
| **HbA1c (Baseline)** | | |
| Number of Subjects (Missing data) | 31 (0) | 31 (0) |
| Mean (Standard Deviation) | 8.27 (0.831) | 8.44 (0.918) |
| Median (Q1, Q3) | 8.40 (7.40, 8.90) | 8.40 (7.70, 8.90) |
| Min, Max | 7.1, 10.3 | 7, 11 |

| **HbA1c (Week 16 of Treatment)** | | |
|---|---|---|
| **Statistical Parameter** | Pharmaceutical Composition 1 Injection (N = 31) | Insulin Degludec/Insulin Aspart Injection (N = 31) |
| Number of Subjects (Missing) | 31 (0) | 31 (0) |
| Mean (Standard Deviation) | 6.83 (0.701) | 7.06 (0.728) |
| Median (Q1, Q3) | 6.90 (6.20, 7.24) | 7.00 (6.50, 7.60) |
| Min, Max | 5.492, 8.8 | 5.8, 8.7 |

| **HbA1c (Week 16 of Treatment - Baseline)** | | |
|---|---|---|
| Number of Subjects (Missing) | 31 (0) | 31 (0) |
| Mean (Standard Deviation) | -1.43 (0.828) | -1.38 (1.103) |
| Median (Q1, Q3) | -1.40 (-1.90, -0.90) | -1.10 (-1.80, -0.70) |
| Min, Max | -3.076, 0.4 | -4.6, 0.6 |

| **Analysis of Change from Baseline in HbA1c After 16 Weeks of Treatment** | | |
|---|---|---|
| Number of Subjects (Missing) | 31 (0) | 31 (0) |
| Least Squares Mean (Standard Error) | -1.50 (0.127) | -1.32 (0.125) |
| Least Squares Mean Difference (95% CI) | -0.19 (-0.54, 0.16) | - |
| P-value | 0.2969 | |

| | | |
|---|---|---|
| Note: Baseline is defined as the last non-missing measurement value before the subject's first dose of the investigational drug. | | |

The Analysis of Covariance (ANCOVA) model will use the change from baseline in HbA1c after 16 weeks of treatment as the dependent variable, with group (categorical variable) as a fixed effect and stratification factors (baseline glycated hemoglobin included as a continuous variable) as covariates.

The p-value refers to the significance test for difference in least squares means between the Compound of Formula (I) injection group and the control group.

### (2) Analysis of Fasting Venous Plasma Glucose

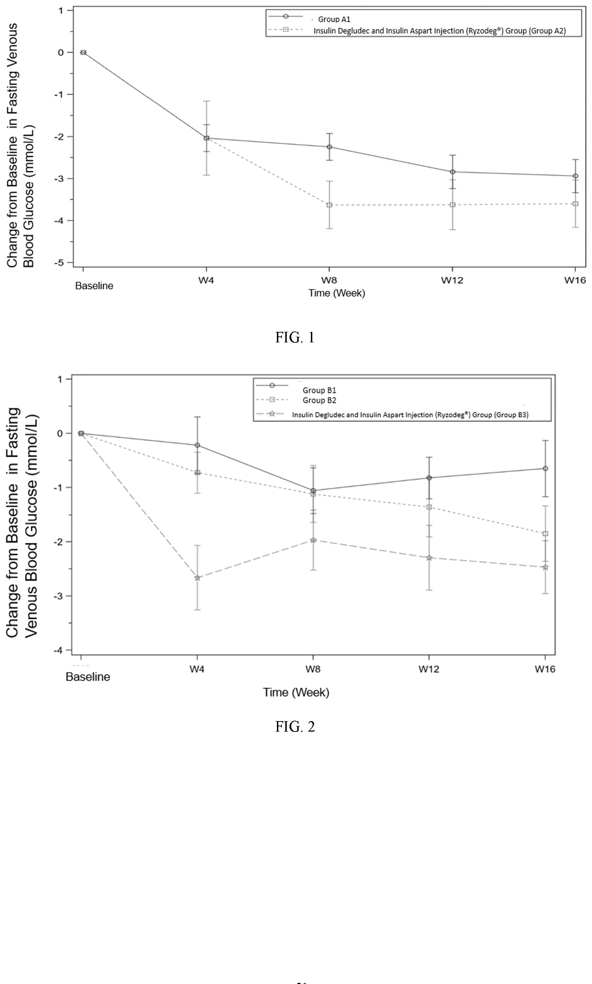

Based on the FAS, an ANCOVA model (with MI imputation) was used to compare the changes from baseline in fasting venous plasma glucose after 16 weeks of treatment between the groups. After 16 weeks of treatment, the least squares mean (standard error) changes from baseline in fasting venous plasma glucose for Group A1 and Group A2 were -3.515 (0.2560) mmol/L and -3.139 (0.2550) mmol/L, respectively. The reduction in fasting venous plasma glucose was greater in Group A1 than in Group A2. A graphical analysis of the change from baseline in fasting venous plasma glucose levels at each visit was performed using an ANCOVA model without imputation; the results are shown in Fig.1.

### (3) Analysis of Proportion of Subjects Achieving HbA1c Reduction

Logistic regression analysis (with MI imputation) was used to compare the proportions of subjects with HbA1c < 7.0% and HbA1c ≤ 6.5% after 16 weeks of treatment between the groups. After 16 weeks of treatment, the proportions of subjects with HbA1c < 7.0% in Group A1 and Group A2 were 53.0% and 48.4%, respectively; the proportions of subjects with HbA1c ≤ 6.5% were 32.8% and 29.0%, respectively. The proportions of subjects achieving HbA1c < 7.0% and HbA1c ≤ 6.5% were both higher in the Compound of Formula (I) injection group than in the Insulin Degludec/Insulin Aspart injection group.

### (4) Reduction in Mean 7-Point Capillary Blood Glucose Levels

Based on the FAS, an ANCOVA model with MI imputation was used to compare the changes from baseline in capillary blood glucose levels from the 7-point SMBG profile (pre-breakfast and 2 hours post-breakfast, pre-lunch and 2 hours post-lunch, pre-dinner and 2 hours post-dinner on the day before the visit, and bedtime on the day before the visit) between the groups. Capillary blood glucose levels at pre-breakfast, 2 hours post-breakfast, pre-lunch, 2 hours post-lunch, pre-dinner, 2 hours post-dinner on the day before the W16 visit, and at bedtime on the day before the visit decreased from baseline in both Group A1 and Group A2. Furthermore, the reductions from baseline in capillary blood glucose at 2 hours post-breakfast and pre-lunch were greater in Group A1 than in Group A2, and the difference in the changes from baseline were statistically significant.

Moreover, after 16 weeks of treatment, the variability of the 7-point capillary blood glucose levels in the Pharmaceutical Composition 1 injection group was lower than thatthe Insulin Degludec/Insulin Aspart injection group, indicating a more stable glycemic control.

**Table 7: 7-Point Capillary Blood Glucose Levels (mmol/L) from Self-Monitored Blood Glucose (SMBG) After 16 Weeks of Treatment**

| Statistical Parameter | Pharmaceutical Composition 1 Injection (N = 31) | Insulin Degludec/Insulin Aspart Injection (N = 31) |
|---|---|---|
| **2 Hours Post-Breakfast on the Day Before the Visit** | | |
| **Change from Baseline in Capillary Blood Glucose at Week 16** | | |
| Number of Subjects (Missing) | 31 (0) | 31 (0) |
| Mean (Standard Deviation) | -5.135 (4.4486) | -4.861 (5.8559) |

| **Analysis of Change from Baseline in Capillary Blood Glucose After 16 Weeks of Treatment** | | |
|---|---|---|
| Least Squares Mean (Standard Error) | -6.093 (0.6484) | -3.901 (0.6601) |
| Least Squares Mean Difference (95% CI) | -2.193 (-3.997, -0.388) | - |
| P-value | 0.0172 | |

| **Pre-Lunch on the Day Before the Visit** | | |
|---|---|---|
| Change from Baseline in Capillary Blood Glucose at Week 16 | | |
| Number of Subjects (Missing) | 30 (1) | 30 (1) |
| Mean (Standard Deviation) | -3.842 (3.5267) | -3.221 (5.2761) |

| **Analysis of Change from Baseline in Capillary Blood Glucose After 16 Weeks of Treatment** | | |
|---|---|---|
| Least Squares Mean (Standard Error) | -4.382 (0.5137) | -2.700 (0.5083) |
| Least Squares Mean Difference (95% CI) | -1.683 (-3.070, -0.295) | - |
| P-value | 0.0175 | |

| | | |
|---|---|---|
| Note: Baseline is defined as the last non-missing measurement value before the subject's first dose of the investigational drug. | | |

The Analysis of Covariance (ANCOVA) model will use the change from baseline in capillary blood glucose levels at each SMBG time point after 16 weeks of treatment as the dependent variable, with group (categorical variable), stratification factor (metformin + other oral antidiabetic drugs vs. metformin), and baseline capillary blood glucose level at each SMBG time point (continuous variable) as explanatory variables.

The p-value refers to the test for difference in least squares means between the treatment group (Pharmaceutical Composition 1 injection) and the Insulin Degludec/Insulin Aspart injection group.

### (5) Daily Dose of Pharmaceutical Composition 1 Injection and Insulin Degludec/Insulin Aspart Injection at Week 16

Based on the FAS, an ANCOVA model was used to compare the doses of Pharmaceutical Composition 1 injection and Insulin Degludec/Insulin Aspart injection used by subjects at Week 16 of treatment (i.e., the last week of treatment). The ANCOVA results for the daily dose of Pharmaceutical Composition 1 injection and Insulin Degludec/Insulin Aspart injection at Week 16 of treatment showed that the adjusted geometric means (standard error) for Group A1 (Pharmaceutical Composition 1 injection in) and Group A2 (Insulin Degludec/Insulin Aspart injection) at Week 16 were 28.17 (1.100) U and 30.75 (1.098) U, respectively. The insulin dose in Group A1 was 8% lower than that in Group A2.

That is, during the last week of treatment, the mean daily insulin dose in the Pharmaceutical Composition 1 injection group was lower than that in the Insulin Degludec/Insulin Aspart injection group.

### (6) Change from Baseline in Body Weight at Each Visit

Based on the FAS, an ANCOVA model was used to compare the changes from baseline in the subjects' body weight after 16 weeks of treatment between the groups. After 16 weeks of treatment, the least squares means (standard error) for the change from baseline in body weight for Group A1 and Group A2 were 1.50 (0.371) kg and 1.75 (0.364) kg, respectively. The magnitude of weight gain in Group A1 was smaller than that in Group A2. The analysis results of the change from baseline in body weight after 16 weeks of treatment showed that the least squares mean difference (95% CI) for the change from baseline between Group A1 and Group A2 was -0.25 (-1.29, 0.79) kg.

### Safety Analysis Results

Safety analysis was based on the SS. Throughout the entire study period, no events of severe hypoglycemic, TEAEs leading to interruption of investigational drug, TEAEs leading to premature withdrawal, or TEAEs leading to death occurred in any group. According to CTCAE grading, the severity of TEAEs was mostly Grade 1-2 (98.4%). The most common TEAEs were hypoglycemia and weight gain, similar to other insulin analogues of the same class.

Overall, the incidence of hypoglycemia in Group A1 was lower than that in Group A2, and the triggers for hypoglycemia were similar between the two groups, mostly without an identifiable trigger, followed by those caused by insufficient food intake.

PK studies demonstrated that the long-acting component, Compound of Formula (I), in Pharmaceutical Composition 1 injection has a prolonged half-life, which contributes to more effective blood glucose reduction, thereby supporting the action of the long-acting product.

The immunogenicity risk of Pharmaceutical Composition 1 injection is low, demonstrating acceptable immunogenicity. Overall, Pharmaceutical Composition 1 injection has a good safety profile.

### Example 2: Clinical Trial of a Pharmaceutical Composition Comprising a Compound of Formula (I) and Insulin Aspart in Combination with Insulin Aspart in Adult Patients with Type 2 Diabetes Inadequately controlled on Prior Basal/Premixed Insulin

### Trial Protocol

This clinical trial is a randomized, double-blind, active-controlled trial designed to evaluate the efficacy, safety, and tolerability of using Pharmaceutical Composition 1 in combination with Insulin Aspart in adult patients with type 2 diabetes inadequately controlled on prior basal or premixed insulin. The medications used are shown in Table 8.

**Table 8: Formulations, Specifications, and Administration Methods of the Investigational Drug and Active Control Drug**

| | Investigational Drug | | Active Control Drug |
|---|---|---|---|
| Formulation Name | Pharmaceutical Composition Comprising Compound (I) and Insulin Aspart | Insulin Aspart Injection (Raplin^{®}) | Insulin Degludec and Insulin Aspart Injection (Ryzodeg^{®}) |
| Specificati on | 3 mL: 180 U (Compound of Formula I: 30 U/mL; Insulin Aspart: 30 U/mL) | 3 mL: 300 U | 3 mL: 300 U |
| Manufacturer | Gan & Lee Pharmaceuticals | Gan & Lee Pharmaceuticals | Novo Nordisk A/S |
| Administra tion Method | Subcutaneous injection (at a site at least approximately two finger-widths away from the periumbilical area of the abdomen) | Subcutaneous injection (at a site at least approximately two finger-widths away from the periumbilical area of the abdomen) | Subcutaneous injection (at a site at least approximately two finger-widths away from the periumbilical area of the abdomen) |

| | | | |
|---|---|---|---|
| Note: Before use, the insulin injection should be equilibrated to room temperature (recommended for approximately 30 minutes). | | | |

### Study Methods

The study population enrolled in this study consists of adult patients with type 2 diabetes inadequately controlled on prior basal/premixed insulin. A multicenter, randomized, open-label, parallel-controlled trial design is employed, aiming to compare the efficacy, safety, and tolerability of Pharmaceutical Composition 1 injection combined with Raplin^{®} versus twice-daily Insulin Degludec/Insulin Aspart injection in patients with type 2 diabetes inadequately controlled on oral antidiabetic drugs combined with basal/premixed insulin (various ratios of commercially available premixed insulin).

The study included a screening period of up to 17 days. Eligible subjects after screening were stratified based on whether metformin was used in combination with other oral antidiabetic drugs at screening (stratification factors: metformin + other oral antidiabetic drugs; metformin) and randomly assigned in a 1:1:1 ratio to either the Pharmaceutical Composition 1 injection combined with once-daily Raplin^{®} group (Group B1), the Pharmaceutical Composition 1 injection combined with twice-daily Raplin^{®} group (Group B2), or the Insulin Degludec/Insulin Aspart injection group (Group B3). The planned enrollment was 90 subjects, with 30 subjects each in Group B1, Group B2, and Group B3. All subjects entered a 16-week treatment period after randomization.

Investigators adjusted the doses of Pharmaceutical Composition 1 injection, Raplin^{®}, or Insulin Degludec/Insulin Aspart injection weekly based on the subject's self-monitored blood glucose (SMBG) results until the fasting blood glucose control target is achieved [i.e., the average of three consecutive pre-breakfast fasting capillary blood glucose measurements is between 4.4 and 6.1 mmol/L (inclusive)]. A 2-week safety follow-up period followed the end of the treatment period.

### Subject Inclusion and Exclusion Criteria

The inclusion criteria for subjects are shown in Table 9. The exclusion criteria for subjects are the same as those described in Example 1.

**Table 9. Subject Inclusion Criteria (Eligible subjects must meet all inclusion criteria)**

| | Limit |
|---|---|
| Inclusion Criteria | 1. Signed the Informed Consent Form (ICF) before the trial, have a full understanding of the trial content, procedures, and possible adverse reactions, and be able to comply with the prohibitions and restrictions specified in this protocol. |
| | 2. Aged between 18 and 75 years (inclusive) at the time of informed consent, male or female. |
| | 3. Have been receiving treatment with up to 3 oral antidiabetic drugs in combination with one basal insulin or premixed insulin before screening (the daily dosing frequency of basal insulin/premixed insulin does not exceed 2 times, and the daily dose is between 10 U and 100 U), and have been on a stable dose of basal insulin/premixed insulin combined with oral antidiabetic drugs for ≥ 3 months. The oral antidiabetic drugs must include metformin (requiring a metformin dose of ≥ 1.5 g or the maximum tolerated dose before screening), and |
| | any concomitant oral antidiabetic drugs are limited to DPP-4 inhibitors (DPP-4i), alpha-glucosidase inhibitors, SGLT2 inhibitors, glucokinase activators, sulfonylureas, and meglitinides. |
| | 4. At screening, female subjects of childbearing potential must have a negative high-sensitivity serum beta-human chorionic gonadotropin (β-hCG) pregnancy test result. |
| | 5. Male or female subjects must agree to use reliable contraception for themselves and their partners within 3 months after the last dose (the contraceptive measures used should comply with local regulations regarding contraceptive methods for subjects participating in clinical studies, see Appendix 2 for details), and must not donate sperm or eggs (ova, oocytes) for the purpose of assisted reproduction. |
| | 6. BMI between 18.5 and 35 kg/m² (inclusive) at screening. |
| | 7. Diagnosis of type 2 diabetes mellitus for ≥ 6 months, according to the 1999 World Health Organization (WHO) diagnostic and classification criteria for diabetes mellitus, and the supplemental diagnostic criteria based on the WHO recommendation on the use of HbA1c in diagnosis (2011). |
| | 8. Mean pre-breakfast fasting capillary blood glucose ≤ 13.9 mmol/L on 3 consecutive days before randomization (including the day of randomization), with 7.0% ≤ HbA1c ≤ 11.0% and fasting venous plasma glucose ≥ 7.0 mmol/L at screening. |

Wherein, the basal insulin includes one or more of insulin glargine, insulin detemir, insulin degludec, and Neutral Protamine Hagedorn (NPH) human insulin, and the premixed insulin includes all marketed premixed insulins.

If a subject meets the inclusion criteria and does not meet any exclusion criteria, the subject will be assigned to a group according to the randomization principle and receive 16 weeks of treatment. The insulin administration methods are shown in Table 8. During the treatment period, the investigator will adjust the insulin dose weekly based on the subject's self-monitored pre-breakfast fasting capillary blood glucose results (three consecutive measurements, including the visit day, starting from two days before the dose administration). The dose adjustment rules are shown in Table 4 and Table 5, with the adjustment target being a average of three consecutive pre-breakfast fasting capillary blood glucose values between 4.4 and 6.1 mmol/L (inclusive).

**Table 10: Insulin Administration Methods During the Treatment Period**

| Gro up | Number of Subjects | Drug | Admin istration Method | Starting Dose | Administration Time |
|---|---|---|---|---|---|
| B1 | 32 | Pharmaceutical Composition containing Compound of Formula (I) and Insulin Aspart + 1 dose of Insulin Aspart | Subcut aneous injection in the abdomen | 70% of the pre-randomization total daily dose of basal/premixed insulin replaced with the Pharmaceutical Composition, and 30% replaced with Insulin Aspart | Pharmaceutical Composition: once daily before breakfast; Insulin Aspart: once daily before dinner |
| B2 | 28 | Pharmaceutical Composition containing Compound of Formula (I) and Insulin Aspart + 2 doses of Insulin Aspart | Subcut aneous injection in the abdomen | 70% of the pre-randomization total daily dose of basal/premixed insulin replaced with the Pharmaceutical Composition, 15% replaced with lunchtime Insulin Aspart, and 15% replaced with dinnertime Insulin Aspart | Pharmaceutical Composition: once daily before breakfast; Insulin Aspart: twice daily, before lunch and before dinner |
| B3 | 31 | Insulin Degludec/Insulin Aspart Injection | Subcut aneous injection in the abdom en | Pre-randomization total daily dose of basal/premixed insulin replaced with an equivalent total dose* | Twice daily, before breakfast and before dinner |

| | | | | | |
|---|---|---|---|---|---|
| Note: *For subjects using once-daily basal/premixed insulin before randomization, it is recommended to equally divide the dose into two daily doses of Insulin Degludec/Insulin Aspart (the specific division is to be determined by the investigator based on the patient's blood glucose control needs), administered once before breakfast and once before dinner. For subjects using twice-daily basal/premixed insulin before randomization, the total daily dose should be equally divided into two daily doses of Insulin Degludec/Insulin Aspart (the specific division is to be determined by the investigator based on the patient's blood glucose control needs), administered once before breakfast and once before dinner. | | | | | |

The weekly dose adjustment rules for the pharmaceutical composition containing the Compound of Formula (I) and Insulin Aspart, and for Insulin Degludec/Insulin Aspart, are shown in Table 11 and Table 12.

**Table 11. Weekly Dose Adjustment Rules for Insulin Degludec/Insulin Aspart and the Pharmaceutical Composition Injection (In the Absence of Hypoglycemic Events*)**

| | |
|---|---|
| Average of 3 Consecutive Pre-meal Capillary Blood Glucose Before Dose Administration | Insulin Dose Adjustment (U/Week) |

| (mmol/L) † | |
|---|---|
| 3.9-4.3 | -2 |
| 4.4-6.1 | No adjustment |
| 6.2-7.0 | +2 |
| 7.1-8.0 | +4 |
| 8.1-9.0 | +6 |
| >9.0 | + 8 |

| | |
|---|---|
| Note: *Refers to instances where the minimum value of three consecutive pre-meal capillary blood glucose measurements before dose administration is ≥ 3.9 mmol/L. The dose adjustment for Insulin Degludec/Insulin Aspart injected before dinner is based on the mean of three consecutive pre-breakfast fasting capillary blood glucose measurements (including the visit day) starting from two days before the dose administration. The dose adjustment for Insulin Degludec/Insulin Aspart injected before breakfast is based on the mean of three consecutive pre-dinner capillary blood glucose measurements (excluding the visit day) starting from three days before the dose administration. The dose adjustment for the Pharmaceutical Composition injection administered before breakfast is based on the mean of three consecutive pre-breakfast fasting capillary blood glucose measurements (including the visit day) starting from two days before the dose administration. If one or more pre-meal capillary blood glucose values are missing, dose adjustment should be based on the remaining SMBG values. If the subject's pre-meal capillary blood glucose fails to reach the predetermined treatment target, the investigator may, depending on the subject's condition, individualize the insulin dose adjustment rules after communication with the sponsor. | |

**Table 12: Weekly Dose Adjustment Rules for Insulin Degludec/Insulin Aspart / Pharmaceutical Composition Injection (In the Presence of Hypoglycemic Events*)**

| Minimum Value of Three Consecutive Pre-Meal Capillary Blood Glucose Measurements Before Dose Administration (mmol/L) | Insulin Dose Adjustment (U/Week) |
|---|---|
| ≤3.0 | -4 (If dose > 45 U, reduce by 10%) |
| 3.1-3.8 | -2 (If dose > 45 U, reduce by 5%) |

| | |
|---|---|
| Note: *Refers to any instance of pre-meal capillary blood glucose < 3.9 mmol/L among the three consecutive measurements before dose administration. The dose adjustment for the Pharmaceutical Composition injection administered before breakfast is based on the minimum value of three consecutive pre-breakfast fasting capillary blood glucose measurements (including the visit day) starting from two days before the dose administration. If a subject experiences pre-meal capillary blood glucose < 3.9 mmol/L on days 1-4 after the dose administration at the weekly visit, or experiences random blood glucose or nocturnal blood glucose < 3.9 mmol/L during the period from each dose administration to before dinner in Group B1, or experiences random blood glucose or nocturnal blood glucose < 3.9 mmol/L during the period from each dose administration to before lunch in Group B2, dose adjustment according to Table 10 is also required. The dose adjustment for Insulin Degludec/Insulin Aspart injected before dinner is based on the minimum value of three consecutive pre-breakfast fasting capillary blood glucose measurements (including the visit day) starting from two days before the dose administration. The dose adjustment for Insulin Degludec/Insulin Aspart injected before breakfast is based on the minimum value of three consecutive pre-dinner capillary blood glucose measurements (excluding the visit day) starting from three days before the dose administration. If a subject experiences pre-breakfast or pre-dinner capillary blood glucose < 3.9 mmol/L on days 1-4 after the dose administration at the weekly visit, or experiences random blood glucose < 3.9 mmol/L at other times after any dose administration, dose adjustment according to Table 10 is also required. This applies unless there is a clear explanation or precipitating factor for the minimum value, such as a missed meal, etc. If the subject's pre-meal capillary blood glucose fails to reach the predetermined treatment target or if hypoglycemic events occur, the investigator may, depending on the subject's condition, individualize the insulin dose adjustment rules after communication with the sponsor. | |

Additionally, the weekly dose adjustment rules for Insulin Aspart are shown in Table 13.

**Table 13 Weekly Dose Adjustment Rules for Insulin Aspart**

| Minimum 2-Hour Postprandial Blood Glucose Over 3 Consecutive Days Before Dose Administration (mmol/L)† | Insulin Aspart Dose Adjustment (U/Week) | |
|---|---|---|
| | If Raplin^{®} Dose < 15 U | If Raplin^{®} Dose ≥ 15 U |
| <6.1 | -2 | -2 |
| 6.1-6.9 | No adjustment | No adjustment |
| 7.0-7.8 | No adjustment | +2 |
| 7.9-10.0 | +2 | +3 |
| ≥ 10.0 | +3 | +4 |

The pre-meal dose of Insulin Aspart is adjusted based on the mean of three consecutive 2-hour postprandial capillary blood glucose measurements (excluding the visit day) starting from three days before the dose administration. If one or more 2-hour postprandial capillary blood glucose values are missing, dose adjustment should be based on the remaining SMBG values. If the subject's bedtime blood glucose is < 4.4 mmol/L, the investigator may reduce the pre-dinner dose of Insulin Aspart based on the subject's condition. If the subject's bedtime blood glucose is ≥ 4.4 mmol/L, but nocturnal blood glucose is < 3.9 mmol/L, the dose of the Pharmaceutical Composition should be reduced according to Table 11. If the subject's 2-hour postprandial capillary blood glucose fails to reach the predetermined treatment target or if hypoglycemic events occur, the investigator may, depending on the subject's condition, individualize the insulin dose adjustment rules after communication with the sponsor.

In addition to the treatment according to Tables 10-13, subjects will concomitantly take metformin and/or other oral drugs. The frequency and dose of metformin and/or other oral drugs taken by the subjects will be the same as their regimen before this trial.

### Analysis Methods

Intention-to-Treat (ITT) Analysis Set: Includes all subjects randomized into the study.

Full Analysis Set (FAS): Based on the ITT principle, includes all subjects who were randomized and had at least one record of study drug administration.

Per Protocol Set (PPS): A subset of the FAS, including all subjects with good compliance during the study period, complete primary efficacy endpoint data, no major protocol deviations affecting the primary efficacy endpoint, and who must have received at least 12 weeks of insulin (study drug) treatment.

Safety Set (SS): Refers to all subjects who were randomized, received at least one dose of the study drug, and had at least one safety assessment.

Pharmacokinetic Analysis Set (PKAS): Includes all subjects who were randomized, received at least one dose of the study drug, had at least one evaluable PK data point, and had no protocol deviations significantly affecting PK evaluation.

Immunogenicity Analysis Set (IS): Includes all subjects who were randomized, received at least one dose of the study drug, and had at least one post-dose immunogenicity assessment.

Analyses of primary and secondary efficacy endpoints, as well as hypoglycemic events, were conducted using the FAS, with supplementary analyses performed using the PPS. Analyses of other safety endpoints were conducted using the SS.

### Study Results

### Subject Disposition

A total of 149 subjects were screened for Part B of this study. The 91 successfully screened subjects were randomly assigned to the Pharmaceutical Composition 1 injection combined with once-daily Raplin^{®} group (Group B1, 32 subjects), the Pharmaceutical Composition 1 injection combined with twice-daily Raplin^{®} group (Group B2, 28 subjects), and the Insulin Degludec/Insulin Aspart injection group (Group B3, 31 subjects). All received the study drug. Except for 6 subjects (6.6%) who voluntarily requested early withdrawal from the trial (3 in Group B1, 2 in Group B2, and 1 in Group B3), the remaining 85 subjects (93.4%) completed the trial according to the protocol.

### Demographic Distribution and Baseline Characteristics

Baseline demographics and diabetes-related baseline characteristics were generally balanced among subjects in Group B1, Group B2, and Group B3.

### Efficacy Results

### (1) Analysis of Glycated Hemoglobin Reduction

Based on the FAS, an Analysis of Covariance (ANCOVA) model [with Multiple Imputation (MI) for missing data] was used to compare the changes from baseline in HbA1c after 16 weeks of treatment between the groups, as shown in Table 14.

After 16 weeks of treatment, the mean (SD) HbA1c values for subjects in Group B1, Group B2, and Group B3 were 6.78 (0.501)%, 6.79 (0.539)%, and 6.78 (0.617)%, respectively. The least squares mean (standard error) changes from baseline were -1.68 (0.093)%, -1.69 (0.102)%, and -1.63 (0.095)%, respectively. Compared with baseline, the reductions in HbA1c in the "Pharmaceutical Composition 1 injection combined with once-daily Raplin^{®} group and the Pharmaceutical Composition 1 injection combined with twice-daily Raplin^{®} group were both greater than that in the Insulin Degludec/Insulin Aspart injection group.

**Table 14: HbA1c (%) Analysis**

| **Statistical Parameter** | **Pharmaceutical Composition 1 Injection + Once-Daily Raplin^{®} (N = 32)** | **Pharmaceutical Composition 1 Injection + Twice-Daily Raplin^{®} (N = 28)** | **Insulin Degludec/Insulin Aspart Injection (N = 31)** |
|---|---|---|---|
| **Analysis of Change from Baseline in HbA1c After 16 Weeks of Treatment** | | | |
| Number of Subjects (Missing) | 32 (0) | 28 (0) | 31 (0) |
| Least Squares Mean (Standard Error) | -1.68 (0.093) | -1.69 (0.102) | -1.63 (0.095) |
| Least Squares Mean Difference (95% CI) | -0.05 (-0.31, 0.21) | -0.06 (-0.33, 0.21) | - |
| P-value | 0.6987 | 0.6510 | |

| | | | |
|---|---|---|---|
| Note: Baseline is defined as the last non-missing measurement value before the subject's first dose of the investigational drug. | | | |

### (2) Analysis of Fasting Venous Plasma Glucose

Based on the FAS, an ANCOVA model (with MI imputation) was used to compare the changes from baseline in fasting venous plasma glucose after 16 weeks of treatment between the groups. A graphical analysis of the change from baseline in fasting venous plasma glucose levels at each visit was performed using an ANCOVA model without imputation; the results are shown in Fig.2.

After 16 weeks of treatment, the mean (SD) fasting venous plasma glucose values for subjects in Group B1, Group B2, and Group B3 were 7.591 (2.1949) mmol/L, 7.463 (1.7462) mmol/L, and 7.269 (2.6958) mmol/L, respectively. The least squares mean (standard error) changes from baseline were -1.422 (0.4423) mmol/L, -1.780 (0.4385) mmol/L, and -2.086 (0.4215) mmol/L, respectively. The reductions in fasting venous plasma glucose in Groups B1 and B2 were both smaller than that in Group B3.

### (3) Analysis of Proportion of Subjects Achieving HbA1c Reduction

Based on the FAS, logistic regression analysis (with MI imputation) was used to compare the proportions of subjects achieving HbA1c < 7.0% and HbA1c ≤ 6.5% after 16 weeks of treatment between the groups.

After 16 weeks of treatment, the proportions of subjects with HbA1c < 7.0% in Group B1, Group B2, and Group B3 were 72.3%, 61.7%, and 59.0%, respectively. The proportion achieving HbA1c < 7% in the Pharmaceutical Composition 1 injection combined with once-daily Raplin^{®} group was higher than that in the Insulin Degludec/Insulin Aspart injection group, while the proportion in the Pharmaceutical Composition 1 injection combined with twice-daily Raplin^{®} group was similar to that of the Insulin Degludec/Insulin Aspart injection group.

After 16 weeks of treatment, the proportions of subjects with HbA1c ≤ 6.5% in Group B1, Group B2, and Group B3 were 28.8%, 41.3%, and 45.5%, respectively. The proportions of subjects achieving HbA1c ≤ 6.5% were similar between Group B2 and Group B3.

### (4) Change from Baseline in Body Weight at Each Visit

Based on the FAS, an ANCOVA model was used to compare the changes from baseline in body weight after 16 weeks of treatment between the groups. The least squares mean difference (95% CI) for the change from baseline between Group B1 and Group B3 was -0.640 (-1.774, 0.493) kg, with no statistically significant difference (P-value = 0.2642). The least squares mean difference (95% CI) for the change from baseline between Group B2 and Group B3 was 0.018 (-1.153, 1.190) kg, with no statistically significant difference (p-value = 0.9750). The results indicate that after 16 weeks of treatment, the magnitude of weight gain in Group B1 was smaller than that in Group B3, while the magnitude of weight gain was similar between Group B2 and Group B3.

### Safety Analysis Results

Safety analysis was based on the SS. Throughout the study period, no TEAEs leading to premature withdrawal or TEAEs leading to death occurred in any group. Pharmaceutical Composition 1 injection demonstrated good safety and tolerability. The majority of TEAEs were Grade 1 in severity. Common TEAEs were hypoglycemia and weight gain, similar to other insulin analogues of the same class.

PK studies indicate that the long-acting component, Compound of Formula (I), in Pharmaceutical Composition 1 injection has a relatively long half-life, which contributes to more effective blood glucose lowering, thereby supporting the action of the long-acting product.

The immunogenicity risk of Pharmaceutical Composition 1 injection is low, demonstrating acceptable immunogenicity.

**Example 3:** Clinical trial of a pharmaceutical composition containing a compound with the structure of formula (I) and insulin aspart in combination with insulin aspart in adult patients with type 2 diabetes mellitus who had a history of inadequately controlled with basal/premixed insulin.

This clinical trial is a randomized, double-blind, positive-controlled study designed to evaluate the efficacy, safety, and tolerability of pharmaceutical composition 1 in combination with insulin aspart in adult patients with type 2 diabetes mellitus who had a history of inadequately controlled with basal/premixed insulin. The initial dosage regimen is shown in Table 15.

**Table 15: Initial Dosage Regimen of the Investigational Drug**

| Group | Num ber of subj ects | Investigational Drug | Adminis tration route | Starting dose |
|---|---|---|---|---|
| C1 | 30 | Before breakfast: Pharmaceutical Composition 1 Injection Before dinner: Rapilin^{®} | Subcuta neous injection in the abdomen | Insulin dosage before breakfast: 70% of the total daily insulin dose before randomization was replaced with the pharmaceutical composition 1 injection; Insulin dosage before dinner: |
| | | | | ① Did not use pre-dinner prandial insulin before randomization: 15% of the total daily insulin dose before randomization wss replaced with pre-dinner Rapilin^{®}. |
| | | | | ② Usedpre-dinner prandial insulin before randomization: replaced with an equivalent dose of pre-dinner Rapilin^{®} according to the original pre-dinner prandial insulin dose. |
| C2 | 30 | Before breakfast: Compound of formula (I)-70 injection (Compound of formula (I) and aspart insulin are administered separately) Before dinner: Rapilin^{®} | Subcuta neous injection in the abdomen | Insulin dosage before breakfast: 49% of the total daily insulin dose before randomization was replaced with compound with the structure of formula (I) injection, and 21% is replaced with pre-breakfast insulin aspart; Insulin dosage before dinner: |
| | | | | ① Did not usepre-dinner prandial insulin before randomization: 15% of the total daily insulin dose before randomization was replaced with pre-dinner Rapilin^{®}. |
| | | | | ② Usedpre-dinner prandial insulin before randomization: replaced with an equivalent dose of pre-dinner Rapilin^{®} according to the original pre-dinner prandial insulin dose. |

The dosage adjustment was identical to Table 4 in Example 1. The inclusion criteria were the same as those in Example 1.

After the full 16-week treatment with the investigational medicinal product was completed, the first day of Week 16 was the final visit, during which subjects were required to return to the research center for relevant safety examinations, etc..

The week following the end of the treatment period served as a safety follow-up period. Investigators might decide to initiate routine treatment based on the subject's overall condition. It was recommended to transition to routine treatment either 2 days after the last study dose or when fasting blood glucose was ≥10 mmol/L. The final decision on the timing of transition to routine treatment rested with the investigator. At the end of the safety follow-up period, subjects underwent procedures to record hypoglycemic events, AE/SAE, and concomitant medications/treatments.

Although certain features of the present invention have been illustrated and described herein, those skilled in the art will now conceive of numerous modifications, substitutions, variations, and equivalents. It should therefore be understood that the appended claims are intended to encompass all such modifications and variations falling within the true scope of the invention.

## Claims

1. A method for treating metabolic syndrome, wherein the method comprises administering a combination of a compound of formula (I) and insulin aspart to a subject in need thereof, wherein a total administered dose of the compound of formula (I) and insulin aspart is about 5U-200U, preferably about 5U-50U, preferably the total doses of each administration are independently the same or different; preferably, when administering the combination of the compound of formula (I) and insulin aspart, the compound of formula (I) and insulin aspart are administered as a mixture or administered separately; preferably, when administering the combination of the compound of formula (I) and insulin aspart, the compound of formula (I) and insulin aspart are administered simultaneously; preferably, the combination of the compound of formula (I) and insulin aspart is a pharmaceutical composition comprising the compound of formula (I) and insulin aspart; 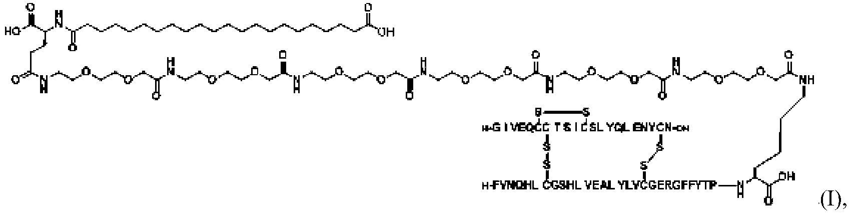 preferably, the metabolic syndrome is diabetes mellitus; preferably, the diabetes is type II diabetes.

2. A method for treating metabolic syndrome, wherein the method comprises administering a pharmaceutical composition comprising a compound of formula (I) and insulin aspart to a subject in need thereof, wherein the total administered dose of the compound of formula (I) and insulin aspart is 5U-200U, preferably about 5U-50U; preferably, the total doses of each administration are independently the same or different; 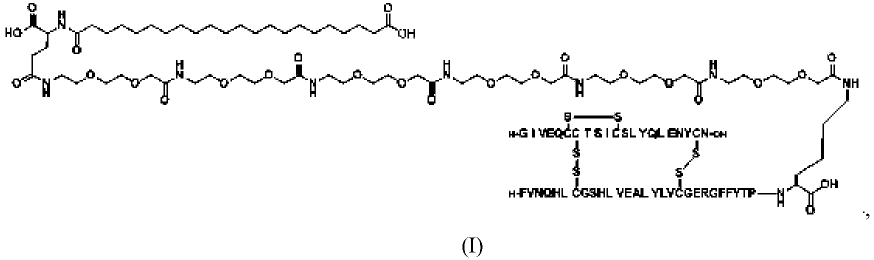 preferably, the metabolic syndrome is diabetes mellitus; preferably, the diabetes mellitus is type II diabetes.

3. The method according to claim 1 or 2, wherein in the combination of the compound of formula (I) and insulin aspart, and in the pharmaceutical composition comprising the compound of formula (I) and insulin aspart, the molar percentage of the compound of formula (I) in the combination or the pharmaceutical composition is about 50%-99%; preferably, the molar percentage of the compound of formula (I) in the combination or the pharmaceutical composition is about 50%, about 55%, about 60%, about 65%, about 70%, about 75%, about 80%, about 85%, about 90%, or about 95%; preferably, the molar percentage of the compound of formula (I) in the combination or the pharmaceutical composition is about 50%, about 60%, about 70%, or about 80%; preferably, a molar ratio of the compound of formula (I) to insulin aspart is 1:1 or 7:3.

4. The method according to any one of claims 1 to 3, wherein a total daily dose of the compound of formula (I) and insulin aspart is about 5U-200U, preferably about 5U-50U, preferably about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 55U, about 60U, about 65U, about 70U, about 75U, about 80U, about 85U, about 90U, about 95U, about 100U, about 110U, about 120U, about 130U, about 140U, about 150U, about 160U, about 170U, about 180U, about 190U, or about 200U;
preferably, the total daily doses are independently the same or different.

5. The method according to any one of claims 1 to 4, wherein the method comprises administering the combination of the compound of formula (I) and insulin aspart or the pharmaceutical composition comprising the compound of formula (I) and insulin aspart to the subject in need thereof at a frequency of once daily or less, preferably the total doses of each administration are independently the same or different.

6. The method according to any one of claims 1 to 5, wherein the method comprises administering the compound of formula (I) and insulin aspart to the subject in need thereof at a frequency of once daily or less and a total dose of about 5U-200U, preferably about 5U-50U, as a starting dose, preferably administering the compound of formula (I) and insulin aspart to the subject in need thereof at a frequency of once daily or less and the following total dose as a starting dose: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 55U, about 60U, about 65U, about 70U, about 75U, about 80U, about 85U, about 90U, about 95U, about 100U, about 110U, about 120U, about 130U, about 140U, about 150U, about 160U, about 170U, about 180U, about 190U, or about 200U;
preferably, for a subject previously administered with a basal or premixed insulin once daily, when switching to administering the combination of the compound of formula (I) and insulin aspart or the pharmaceutical composition comprising the compound of formula (I) and insulin aspart, a total starting dose of the compound of formula (I) and insulin aspart is about 50%-90%, preferably about 60%-85%, preferably about 70%-80%, preferably about 50%, about 60%, about 70%, about 75%, or about 80% of the total daily dose of the previous basal or premixed insulin.

7. The method according to any one of claims 1 to 6, wherein the method further comprises, after administering the compound of formula (I) and insulin aspart to the subject in need thereof at the starting dose, further administering the compound of formula (I) and insulin aspart at a frequency of once daily or less, wherein a total dose of each administration is the same as or different from the starting dose, preferably, the total dose of each administration is determined according to the individual conditions of the subject.

8. The method according to any one of claims 1 to 7, wherein the method comprises, when administering the combination of the compound of formula (I) and insulin aspart, or the pharmaceutical composition comprising the compound of formula (I) and insulin aspart, the compound of formula (I) and insulin aspart are administered to the subject in need thereof at a frequency of once daily or less and at a total dose of about 5U-200U, preferably about 5U-50U, as the starting dose; preferably the compound of formula (I) and insulin aspart are administered to the subject in need thereof at a frequency of once daily or less and the following total dose is used as the starting dose: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 55U, about 60U, about 65U, about 70U, about 75U, about 80U, about 85U, about 90U, about 95U, about 100U, about 110U, about 120U, about 130U, about 140U, about 150U, about 160U, about 170U, about 180U, about 190U, or about 200U;
after administering the starting dose for 1 day, 2 days, 3 days, or 4 days, or after administering for 1, 2, 3, 4, 5, 6, 7, 8, 9, 10, 11, 12, 13, 14, 15 or 16 weeks, the compound of formula (I) and insulin aspart are administered at a frequency of once daily and at a total dose of M, wherein the dose M administered each day is the same or different, and is independently about 1U-200U, preferably about 1U-70U, preferably is independently about 1U, about 2U, about 3U, about 4U, about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 55U, about 60U, about 65U, about 70U, about 75U, about 80U, about 85U, about 90U, about 95U, about 100U, about 110U, about 120U, about 130U, about 140U, about 150U, about 160U, about 170U, about 180U, about 190U, or about 200U.

9. The method according to any one of claims 1 to 8, wherein the method comprises administering the compound of formula (I) and insulin aspart to the subject in need thereof at a total dose of about 5U-200U as the starting dose and at a frequency of once daily or less, preferably administering the compound of formula (I) and insulin aspart to the subject in need thereof at a frequency of once daily or less and at a total dose of about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 55U, about 60U, about 65U, about 70U, about 75U, about 80U, about 85U, about 90U, about 95U, about 100U, about 110U, about 120U, about 130U, about 140U, about 150U, about 160U, about 170U, about 180U, about 190U, or about 200U;
after administering the starting dose for 1 day, 2 days, 3 days or 4 days, or after administering for 1, 2, 3, 4, 5, 6, 7, 8, 9, 10, 11, 12, 13, 14, 15 or 16 weeks, the daily dose M is adjusted daily, weekly, or more frequently.

10. The method according to claim 8 or 9, wherein the daily dose M is adjusted daily, weekly or more frequently according to the following rules:
a) when a fasting blood glucose value of the subject on the day of administration before dosing is 3.9-4.3mmol/L, or when an average of three consecutive prebreakfast fasting capillary blood glucose values of the subject from two or three days before administration is 3.9-4.3mmol/L, the dose M is adjusted to be reduced by about 1U-6U relative to the previous dose, preferably about 1U-4U, preferably about 1U, about 2U, about 3U, or about 4U relative to the previous dose; or
b) when the fasting blood glucose value of the subject on the day of administration before dosing is 4.4-6.1mmol/L, or when the average of three consecutive fasting capillary blood glucose values of the subject before breakfast from two days or three days before administration is 4.4-6. 1mmol/L, the dose M is the previous dose; or
c) when the fasting blood glucose value of the subject on the day of administration before dosing is 6.2-7.0mmol/L, or when the average of three consecutive prebreakfast fasting capillary blood glucose values of the subject from two or three days before administration is 6.2-7.0mmol/L, the dose M is adjusted to be increased by about 1U-4U relative to the previous dose, preferably about 1U, about 2U, about 3U or about 4U relative to the previous dose; or
d) when the fasting blood glucose value of the subject on the day of administration before dosing is 7.1-8.0mmol/L, or when the average of three consecutive prebreakfast fasting capillary blood glucose values of the subject from two or three days before administration is 7.1-8.0mmol/L, the dose M is adjusted to be increased by about 2U-6U relative to the previous dose, preferably about 3U, about 4U, about 5U or about 6U relative to the previous dose; or
e) when the fasting blood glucose value of the subject on the day of administration before dosing is 8.1-9.0mmol/L, or when the average of three consecutive prebreakfast fasting capillary blood glucose values of the subject from two or three days before administration is 8.1-9.0mmol/L, the dose M is adjusted to be increased by about 4U-8U relative to the previous dose, preferably about 5U, about 6U, about 7U or about 8U relative to the previous dose; or
f) when the fasting blood glucose value of the subject on the day of administration before dosing is > 9.0mmol/L, or when the average of three consecutive prebreakfast fasting capillary blood glucose values of the subject from two or three days before administration is > 9.0mmol/L, the dose M is adjusted to be increased by about 6U-10U relative to the previous dose, preferably about 7U, about 8U, about 9U or about 10U relative to the previous dose; or
g) when the fasting blood glucose value of the subject on the day of administration before dosing is ≤ 3.0mmol/L, or when the minimum value of three consecutive prebreakfast fasting capillary blood glucose values of the subject from two or three days before administration is ≤ 3.0mmol/L, the dose M is adjusted to be reduced by about 1U-8U relative to the previous dose, preferably by about 2U-6U, preferably by about 3U, about 4U or about 5U relative to the previous dose, or when the previous dose is > 45U, the dose M is adjusted to be reduced by 5% to 15% relative to the previous dose, preferably by 10%; or
h) when the fasting blood glucose value of the subject on the day of administration before dosing is 3.1-3.8mmol/L, or when the minimum value of three consecutive prebreakfast fasting capillary blood glucose values of the subject from two days or three days before administration is 3.1-3.8mmol/L, the dose M is adjusted to be reduced by about 1U-4U relative to the previous dose, preferably by about 1U-3U, preferably by about 1U, about 2U or about 3U relative to the previous dose, or when the previous dose is > 45U, the dose M is adjusted to be reduced by 2% to 10%, preferably by 5% relative to the previous dose.

11. The method according to any one of claims 1 to 10, wherein the subject is a patient with type II diabetes inadequately controlled by oral antidiabetic drugs; or the subject is a patient with diabetes inadequately controlled by previous use of a basal insulin or premixed insulin; or the subject is a patient with type II diabetes inadequately controlled by previous use of a basal insulin or premixed insulin; or the subject is a patient with type II diabetes inadequately controlled by previous use of a basal insulin once daily or a premixed insulin at least once daily, with or without a mealtime insulin before dinner.

12. The method according to any one of claims 1 to 11, wherein the subject:
has a BMI between 10 kg/m² and 50 kg/m², preferably 18.5-35 kg/m²; and/or
has an HbA1c of 7.5% to 11.0%; and/or
has a fasting venous blood glucose of ≥ 7.0 mmol/L; and/or
has received one or more oral antidiabetic drugs, preferably at most three oral antidiabetic drugs, and the oral antidiabetic drugs include metformin, and further include a combination with or without an oral antidiabetic drug selected from the group consisting of DPP-4 inhibitor, α-glycosidase inhibitor, SGLT2 inhibitor, and glucokinase activator .

13. The method according to any one of claims 1 to 12, wherein the combination of the compound of formula (I) and insulin aspart, or the pharmaceutical composition comprising the compound of formula (I) and insulin aspart is administered at a fixed or non - fixed time before any meal, preferably before breakfast.

14. The method according to any one of claims 1 to 13, wherein the combination of the compound of formula (I) and insulin aspart, or the pharmaceutical composition comprising the compound of formula (I) and insulin aspart is administered by subcutaneous injection, preferably by abdominal subcutaneous injection.

15. The method according to any one of claims 1 to 14, wherein the combination of the compound of formula (I) and insulin aspart, or the pharmaceutical composition comprising the compound of formula (I) and insulin aspart is for long-term administration, preferably for at least 4 weeks, preferably for at least 8 weeks, preferably for at least 16 weeks, preferably for at least 20 weeks, preferably for at least 25 weeks, preferably for at least 32 weeks.

16. The method according to any one of claims 1 to 15, wherein when the subject is a patient with diabetes inadequately controlled by previous use of a basal insulin or premixed insulin, the method further comprises administering insulin aspart alone to the subject, preferably before dinner daily.

17. The method according to claim 16, wherein the method comprises administering the compound of formula (I) and insulin aspart to the subject in need thereof at a total dose of 50%-100% of the previous total daily dose of the basal and/or premixed insulin as a starting dose at a frequency of once daily or less; and/or
further, comprises administering insulin aspart at a total dose of 0%-50% of the previous total daily dose of the basal and/or premixed insulin as the starting dose at a frequency of once daily or twice daily;
wherein, the compound of formula (I) and insulin aspart are administered at a first time point, the insulin aspart is administered at a second time point, and/or the insulin aspart is administered at the second time point and/or a third time point;
preferably, the method comprises administering the compound of formula (I) and the insulin aspart to the subject in need thereof at the first time point with 60%, 70%, 80%, 90% or 100% of the previous total daily dose of the basal and/or premixed insulin as the starting dose at a frequency of once daily or less; and/or
further, comprises administering the insulin aspart once daily or twice daily at the second time point and/or the third time point with 5%, 10%, 15%, 20% or 40% of the previous total daily dose of the basal and/or premixed insulin as the starting dose.

18. The method according to claim 17, wherein the method comprises administering the compound of formula (I) and the insulin aspart at a total dose of N and at a frequency of once daily, and/or further administering insulin aspart at a dose of O and at a frequency of once daily or twice daily, after the starting dose is administered for 1 week, wherein the dose N administered each day is the same or different, and/or the dose O administered each day is the same or different; wherein N and O are each independently 0%-100% of the previous total daily dose of the basal and/or premixed insulin, preferably each independently 5%, 10%, 15%, 20%, 25%, 30%, 35%, 40%, 45%, 50%, 55%, 60%, 65%, 70%, 75%, 80%, 85%, 90%, 95% or 100% of the previous total daily dose of the basal and/or premixed insulin.

19. The method according to claim 17 or 18, wherein the method comprises administering the compound of formula (I) and the insulin aspart at a total dose of N and at a frequency of once daily after the starting dose is administered for 1 week, and/or further administering insulin aspart at a dose of O and at a frequency of once daily or twice daily, wherein the dose N administered each day is the same or different, and/or the dose O administered each day is the same or different; wherein N and O are each independently 0%-100% of the previous total daily dose of the basal and/or premixed insulin, preferably each independently 5%, 10%, 15%, 20%, 25%, 30%, 35%, 40%, 45%, 50%, 55%, 60%, 65%, 70%, 75%, 80%, 85%, 90%, 95% or 100% of the previous total daily dose of the basal and/or premixed insulin;
after administering the starting dose for 1, 2, 3, 4, 5, 6, 7, 8, 9, 10, 11, 12, 13, 14, 15, or 16 weeks, the doses N and O administered each day are adjusted at a frequency of once weekly or more frequently.

20. The method according to claim 18 or 19, wherein the dose N administered each day is adjusted at a frequency of once weekly or more frequently according to the following rules:
a) when a fasting blood glucose value of the subject on the day of administration before dosing is 3.9-4.3mmol/L, or when an average of three consecutive pre-meal capillary blood glucose values of the subject before administration is 3.9-4.3mmol/L, the dose N is adjusted to be reduced by about 1U-6U relative to the previous dose, preferably about 1U-4U, preferably about 1U, about 2U, about 3U or about 4U relative to the previous dose; or
b) when the fasting blood glucose value of the subject on the day of administration before dosing is 4.4-6.1mmol/L, or when the average of three consecutive pre-meal capillary blood glucose values of the subject before administration is 4.4-6.1mmol/L, the dose N is the previous dose; or
c) when the fasting blood glucose value of the subject on the day of administration before dosing is 6.2-7.0mmol/L, or when the average of three consecutive pre-meal capillary blood glucose values of the subject before administration is 6.2-7.0mmol/L, the dose N is adjusted to be increased by about 1U-4U relative to the previous dose, preferably about 1U, about 2U, about 3U or about 4U relative to the previous dose; or
d) when the fasting blood glucose value of the subject on the day of administration before dosing is 7.1-8.0mmol/L, or when the average of three consecutive pre-meal capillary blood glucose values of the subject before administration is 7.1-8.0mmol/L, the dose N is adjusted to be increased by about 2U-6U relative to the previous dose, preferably about 3U, about 4U, about 5U or about 6U relative to the previous dose; or
e) when the fasting blood glucose value of the subject on the day of administration before dosing is 8.1-9.0mmol/L, or when the average of three consecutive pre-meal capillary blood glucose values of the subject before administration is 8.1-9.0mmol/L, the dose N is adjusted to be increased by about 4U-8U relative to the previous dose, preferably about 5U, about 6U, about 7U or about 8U relative to the previous dose; or
f) when the fasting blood glucose value of the subject on the day of administration before dosing is > 9.0mmol/L, or when the average of three consecutive pre-meal capillary blood glucose values of the subject before administration is > 9.0mmol/L, the dose N is adjusted to be increased by about 6U-10U relative to the previous dose, preferably about 7U, about 8U, about 9U or about 10U relative to the previous dose; or
g) when the fasting blood glucose value of the subject on the day of administration before dosing is ≤ 3.0mmol/L, or when the minimum value of three consecutive pre-meal capillary blood glucose values of the subject before administration is ≤ 3.0mmol/L, the dose N is adjusted to be reduced by about 1U-8U relative to the previous dose, preferably by about 2U-6U, preferably by about 3U, about 4U or about 5U relative to the previous dose, or when the previous dose is > 45U, the dose M is adjusted to be reduced by 5% to 15% relative to the previous dose, preferably by 10%; or
h) when the fasting blood glucose value of the subject on the day of administration before dosing is 3.1-3.8mmol/L, or when the minimum value of three consecutive pre-meal capillary blood glucose values of the subject before administration is 3.1-3.8mmol/L, the dose N is adjusted to be reduced by 1U-4U relative to the previous dose, preferably by 1U-3U, preferably by 1U, 2U or 3U relative to the previous dose, or when the previous dose is > 45U, the dose N is adjusted to be reduced by 2% to 10%, preferably by 5% relative to the previous dose.

21. The method according to claim 18 or 19, wherein the dose O administered each day is adjusted at a frequency of once weekly or more frequently according to the following rules:
a) when the subject's minimum 2-hour postprandial blood glucose on three consecutive occasions before administration is less than 6.1mmol/L, the dose O is adjusted to be reduced by about 1U-4U relative to the previous dose, preferably by about 1U, about 2U or about 3U; or
b) when the subject's minimum 2-hour postprandial blood glucose on three consecutive occasions before administration is 6.1-6.9mmol/L, the dose O is the previous dose; or
c) when the subject's minimum 2-hour postprandial blood glucose on three consecutive occasions before administration is 7.0- 7.8mmol/L, the dose O is adjusted to be increased by about 0U-4U relative to the previous dose, preferably by about 0U, about 1U, about 2U or about 3U; or
d) when the subject's minimum 2-hour postprandial blood glucose on three consecutive occasions before administration is 7.9- 10.0mmol/L, the dose O is adjusted to be increased by about 0U-4U relative to the previous dose, preferably by about 0U, about 1U, about 2U or about 3U; or
e) when the subject's minimum 2 - hour postprandial blood glucose on three consecutive occasions before administration is ≥ 10.0mmol/L, the dose O is adjusted to be increased by about 2U - 5U relative to the previous dose, preferably by about 2U, about 3U, about 4U or about 5U.

22. The method according to claim 21, wherein the adjustment rules are further adjusted according to the previous dose of the insulin aspart,
when the dose of insulin aspart is less than 15U:
a) when the subject's minimum 2-hour postprandial blood glucose on three consecutive occasions before administration is less than 6.1mmol/L, the dose O is adjusted to be reduced by about 1U-4U relative to the previous dose, preferably by about 1U, about 2U or about 3U; or
b) when the subject's minimum 2-hour postprandial blood glucose on three consecutive occasions before administration is 6.1-7.8 mmol/L, the dose O is the previous dose; or
c) when the subject's minimum 2-hour postprandial blood glucose on three consecutive occasions before administration is 7.9- 10.0mmol/L, the dose O is adjusted to be increased by about 1U-4U relative to the previous dose, preferably by about 1U, about 2U or about 3U; or
d) when the subject's minimum 2 - hour postprandial blood glucose on three consecutive occasions before administration is ≥ 10.0mmol/L, the dose O is adjusted to be increased by about 2U - 4U relative to the previous dose, preferably by about 2U, about 3U or about 4U; or
when the dose of insulin aspart is ≥ 15U:
a) when the subject's minimum 2-hour postprandial blood glucose on three consecutive occasions before administration is less than 6.1mmol/L, the dose O is adjusted to be reduced by about 1U-4U relative to the previous dose, preferably by about 1U, about 2U or about 3U; or
b) when the subject's minimum 2-hour postprandial blood glucose on three consecutive occasions before administration is 6.1-6.9 mmol/L, the dose O is the previous dose; or
c) when the subject's minimum 2-hour postprandial blood glucose on three consecutive occasions before administration is 7.0-7.8 mmol/L, the dose O is adjusted to be increased by about 1U-3U relative to the previous dose, preferably by about 1U, about 2U or about 3U; or
d) when the subject's minimum 2 - hour postprandial blood glucose on three consecutive occasions before administration is 7.9-10.0mmol/L, the dose O is adjusted to be increased by about 2U - 4U relative to the previous dose, preferably by about 2U, about 3U or about 4U; or
e) when the subject's minimum 2 - hour postprandial blood glucose on three consecutive occasions before administration is ≥ 10.0mmol/L, the dose O is adjusted to be increased by about 3U - 5U relative to the previous dose, preferably by about 3U, about 4U or about 5U.

23. The method according to any one of claims 16 to 22, wherein the first time point, the second time point and the third time point are each independently and optionally selected from before breakfast, before lunch and before dinner; preferably, the first time point is before breakfast, the second time point is before dinner, and the third time point is before lunch.

24. The method according to any one of claims 16 to 23, wherein the subject:
has a BMI between 10 kg/m² and 50 kg/m², preferably between 18.5 kg/m² and 35 kg/m²; and/or
has an HbA1c of 7.5% to 11.0%; and/or
has a fasting venous blood glucose of ≥ 7.0 mmol/L; and/or
has received one or more oral antidiabetic drugs, preferably has received a treatment of at most three oral antidiabetic drugs combined with one basal insulin or premixed insulin, wherein the dosing frequency of the basal insulin or premixed insulin is no more than twice daily, the daily dose is about 10U - 100U, and the duration of the treatment with basal insulin or premixed insulin combined with a stable dose of oral antidiabetic drugs is ≥ 3 months.

25. The method according to claim 24, wherein the oral antidiabetic drugs include metformin, which further combines or does not combine with an oral antidiabetic drug selected from the group consisting of DPP4 inhibitor (DPP-4i), α-glycosidase inhibitor, SGLT2 inhibitor, glucokinase activator, sulfonylureas, and glinides.

26. The method according to any one of claims 16 to 25, wherein the pharmaceutical composition is administered via subcutaneous injection, preferably via abdominal subcutaneous injection.

27. The method according to any one of claims 16 to 26, wherein the pharmaceutical composition combined with insulin aspart is for a long-term administration, preferably for at least 4 weeks, preferably for at least 8 weeks, preferably for at least 16 weeks, preferably for at least 20 weeks, preferably for at least 25 weeks, preferably for at least 32 weeks.

28. The method according to any one of claims 1 to 27, wherein the subject further receives daily oral administration of metformin.

29. The method according to any one of claims 1 to 28, wherein in the pharmaceutical composition comprising the compound of formula (I) and insulin aspart, the content of the compound of formula (I) is about 20-200U/ml, preferably about 30-100U/ml, preferably about 30U/ml, about 40U/ml, about 50U/ml, about 60U/ml, about 70U/ml, about 80U/ml, about 90U/ml, about 100U/ml, preferably about 30U/ml, about 40U/ml, about 50U/ml, about 60U/ml, or about 70U/ml.

30. The method according to any one of claims 1 to 29, wherein the pharmaceutical composition comprising the compound of formula (I) and insulin aspart further comprises 10-25mg/ml glycerol, 1.5-6mg/ml phenol, 0.5-3mg/ml m-cresol, 5-50 µg/ml zinc ions and 0.5-2.0mg/ml sodium chloride, and the pH of the composition is 7.0-8.0;
preferably, the pharmaceutical composition comprising the compound of formula (I) and insulin aspart further comprises 10-25mg/ml glycerol, 1.5-3.5mg/ml phenol, 0.5-2.0mg/ml m-cresol, 5-50µg/ml zinc ions and 0.5-2.0mg/ml sodium chloride, and the pH of the composition is 7.0-8.0;
preferably, the pharmaceutical composition comprising the compound of formula (I) and insulin aspart further comprises 17mg/ml glycerol, 2.82mg/ml phenol, 1.08mg/ml m-cresol, 15-40 µg/ml zinc ions and 1.17mg/ml sodium chloride, and the pH of the composition is 7.2-7.8;
preferably, the pharmaceutical composition comprises 0.18mM of the compound of formula (I), 0.18mM insulin aspart, 17mg/ml glycerol, 2.82mg/ml phenol, 1.08mg/ml m-cresol, 18.75 µg/ml zinc ions and 1.17mg/ml sodium chloride, and the pH of the composition is 7.2-7.8;
or the pharmaceutical composition comprises 0.42mM of the compound of formula (I), 0.18mM insulin aspart, 17mg/ml glycerol, 2.82mg/ml phenol, 1.08mg/ml m-cresol, 35.9 µg/ml zinc ions and 1.17mg/ml sodium chloride, and the pH of the composition is 7.2-7.8;
or the pharmaceutical composition comprises 0.3mM of the compound of formula (I), 0.3mM insulin aspart, 17mg/ml glycerol, 2.82mg/ml phenol, 1.08mg/ml m-cresol, 31.25 µg/ml zinc ions and 1.17mg/ml sodium chloride, and the pH of the composition is 7.2-7.8.

31. A method for treating metabolic syndrome, wherein the method comprises administering to a subject in need thereof a compound of formula (I), or a pharmaceutically acceptable salt, amide or ester thereof, 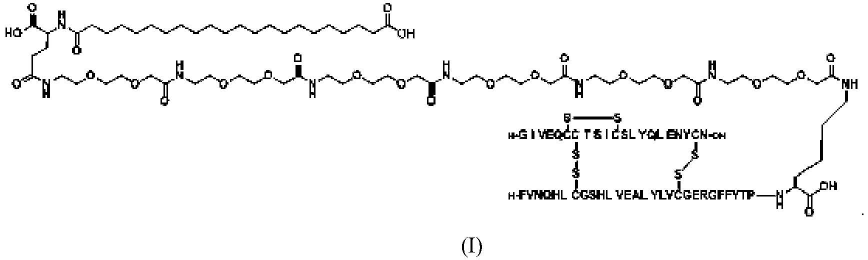
preferably, the metabolic syndrome is diabetes mellitus; preferably, the diabetes mellitus is type I diabetes or type II diabetes;
preferably, the subject is a patient with diabetes inadequately controlled by oral antidiabetic drugs, preferably a patient with type II diabetes;
preferably, the subject is a patient with diabetes inadequately controlled by previous use of a basal insulin or premixed insulin, preferably a patient with type II diabetes.

32. The method according to claim 31, wherein a single dose of the compound of formula (I) is calculated according to the body weight of the subject, and the single dose is 0.2-30nmol/kg, preferably 0.45nmol/kg, 0.9nmol/kg, 1.8 nmol/kg, 3.6nmol/kg, 7.2nmol/kg, 14.4nmol/kg or 28.8nmol/kg.

33. The method according to claim 32, wherein the single dose of the compound of formula (I) is calculated according to the body weight of the subject, and the single dose is 0.03-5U/kg, preferably about 0.075U/kg, about 0.15U/kg, about 0.3U/kg, about 0.6U/kg, about 1.2U/kg, about 2.4U/kg or about 4.8U/kg.

34. The method according to any one of claims 1 to 33, **characterized in that** the subject achieves a reduction in glycated hemoglobin by at least 0.1% after administering the combination or the pharmaceutical composition of the compound of formula (I) and insulin aspart relative to that before administration, preferably by at least 0.2%, preferably by at least 0.3%, preferably by at least 0.4%, preferably by at least 0.5%, preferably by 0.5%-30%, preferably by 0.6%-25%, preferably by 0.62%, 0.76%, 0.9%, 1%, 2%, 3%, 4%, 5%, 6%, 6.5%, 7%, 8%, 9%, 10%, 11%, 12%, 13%, 13.5%, 14%, 15%, 16%, 17%, 18%, 18.6%, 19%, or 20%; preferably, the subject achieves a reduction in glycated hemoglobin by at least 0.1% after administering the combination or the pharmaceutical composition of the compound of formula (I) and insulin aspart for 16 weeks, 20 weeks, 25 weeks, 30 weeks, or 35 weeks, preferably by at least 0.2%, preferably by at least 0.3%, preferably by at least 0.4%, preferably by at least 0.5%, preferably by 0.5%-30%, preferably by 0.6%-25%, preferably by 0.62%, 0.76%, 0.9%, 1%, 2%, 3%, 4%, 5%, 6.5%, 7%, 8%, 9%, 10%, 11%, 12%, 13%, 13.5%, 14%, 15%, 16%, 17%, 18%, 18.6%, 19%, or 20%.

35. A kit, comprising:
a compound of formula (I), or a pharmaceutically acceptable salt thereof, 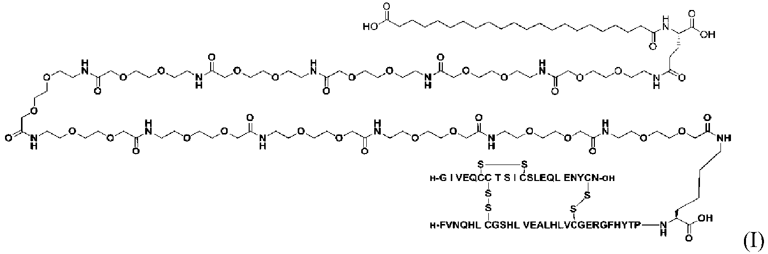
a packaging material, and
a label or package insert contained in the packaging material, the label or package insert providing instructions for treating a subject with the compound of formula (I), or the pharmaceutically acceptable salt thereof, according to the method of any one of claims 1 to 34.
